(19) Europäisches Patentamt
European Patent Office
Office européen des brevets

(11) **EP 3 288 895 B1**

(12) **EUROPÄISCHE PATENTSCHRIFT**

(45) Veröffentlichungstag und Bekanntmachung des Hinweises auf die Patenterteilung:
**26.09.2018 Patentblatt 2018/39**

(21) Anmeldenummer: **16718643.6**

(22) Anmeldetag: **21.04.2016**

(51) Int Cl.:
**C01B 33/152** (2006.01)   **C01B 33/155** (2006.01)
**C01B 33/158** (2006.01)

(86) Internationale Anmeldenummer:
**PCT/EP2016/058833**

(87) Internationale Veröffentlichungsnummer:
**WO 2016/173911 (03.11.2016 Gazette 2016/44)**

(54) **VERFAHREN ZUR HERSTELLUNG ORGANISCH MODIFIZIERTER AEROGELE**

PROCESS FOR PRODUCING ORGANICALLY MODIFIED AEROGELS

PROCÉDÉ DE FABRICATION D'AÉROGELS MODIFIÉS PAR DES GROUPES ORGANIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **29.04.2015 DE 102015207939**

(43) Veröffentlichungstag der Anmeldung:
**07.03.2018 Patentblatt 2018/10**

(73) Patentinhaber: **Wacker Chemie AG**
**81737 München (DE)**

(72) Erfinder:
• **HINDELANG, Konrad**
**80939 München (DE)**
• **GOTTSCHALK-GAUDIG, Torsten**
**84561 Mehring (DE)**
• **JANTKE, Dominik**
**85386 Eching (DE)**
• **WEIDNER, Richard**
**84489 Burghausen (DE)**

(74) Vertreter: **Fritz, Helmut et al**
**Wacker Chemie AG**
**Zentralbereich Patente, Marken und Lizenzen**
**Hanns-Seidel-Platz 4**
**81737 München (DE)**

(56) Entgegenhaltungen:
EP-B1- 0 963 347    US-A- 2 945 817
US-A- 5 789 495    US-A1- 2006 281 828

EP 3 288 895 B1

**Beschreibung**

Gegenstand der Erfindung ist ein Verfahren zur Herstellung organisch modifizierter Aerogele, indem ein Sol enthaltend $[SiO_{4/2}]$ -Einheiten und $[R_xSiO_{(4-x)/2}]$ -Einheiten hergestellt wird, wobei bereits bei der Herstellung des Sols ein Phasenvermittler zugegeben oder ein Phasenvermittler aus dem Sol gebildet wird, aus dem Sol ein Gel gebildet wird, wobei der Gehalt an Phasenvermittler in der Porenflüssigkeit zwischen 1 und 50 Gew.-% liegt, das erhaltene Gel in Anwesenheit von über 0,1 Gew.-% des Phasenvermittlers in einem Gemisch enthaltend ein Disiloxan der Formel $R_3Si-O-SiR_3$ und Initiator oberflächenmodifiziert wird, wobei das Gemisch mindestens 20 Gew.-% des Disiloxans der Formel $R_3Si-O-SiR_3$ enthält und wobei der Initiator aus Säure oder Chlorsilan oder Gemischen daraus besteht und die erhaltenen Gele getrocknet werden, wobei X gleich oder verschieden sein kann und 1, 2 oder 3 ist und wobei R gleich oder verschieden sein kann und Wasserstoff oder ein organischer, substituierter oder unsubstituierter Rest ist.

Aerogele sind hochporöse Festkörper die bis zu mehr als 95 % des Volumens aus Poren bestehen. Während ein Lyogel ein mit Flüssigkeit gefülltes Gerüst darstellt, sind die Poren des Aerogels mit Luft gefüllt. Bei einem Hydrogel, das einen Spezialfall des Lyogels darstellt, besteht die Porenflüssigkeit zu mindestens 50% aus Wasser. Durch die poröse Struktur besitzen Aerogele eine hohe spezifische Oberfläche, geringe Porendurchmesser und eine besonders geringe Dichte. Diese Eigenschaften machen Aerogele zu idealen Materialien für Anwendungen in der thermischen Isolierung.

Es gibt verschiedene Arten von Aerogelen, wobei solche auf Silicatbasis am verbreitetsten und technisch aufgrund ihrer geringen Brennbarkeit von besonderer Relevanz sind.

Aerogele weisen eine Verästelung von Partikelketten mit sehr vielen Zwischenräumen in Form von offenen Poren auf. Diese Ketten besitzen Kontaktstellen, so dass sich letztendlich das Bild eines stabilen, schwammartigen Netzes ergibt.

Die Herstellung eines Aerogels verläuft im Prinzip sehr einfach. In einem ersten Schritt wird ein entsprechendes Lyogel hergestellt, wobei in einem zweiten Schritt das Lösungsmittel durch Luft ersetzt wird, d.h. es wird getrocknet. Die Trocknung, d.h. Entfernung der Porenflüssigkeit, ist der für die Qualität von Aerogelen wesentliche Prozessschritt. Hierbei muss die Zerstörung der Gelstruktur vermieden werden. Hierfür existieren zwei wesentliche Strategien:

1) Durch "überkritisches Trocknen", d.h. Druck- und Temperaturbedingungen oberhalb des kritischen Punktes, kann erreicht werden, dass das Gel seine Struktur behält und nicht schrumpft oder kollabiert. Kapillarkräfte und damit die Zerstörung des Netzwerks werden im überkritischen Bereich weitgehend vermieden. Der Nachteil dieser Methode ist, dass für dieses Verfahren eine technisch anspruchsvolle, kostspielige Hochdruck-Technologie erforderlich ist und das Verfahren deshalb großtechnisch, insbesondere als kontinuierliches Verfahren, schwer zu realisieren ist.
2) Das gleiche Ergebnis kann durch Trocknen bei Normaldruck erreicht werden, wenn zuvor die Porenoberfläche durch Modifizierung (Silylierung) passiviert wurde. Dies bedeutet, dass durch Silylierung der freien Silanol-Gruppen im Gel die irreversible Schrumpfung der Gelstruktur während der Trocknung weitgehend vermieden werden kann. Da für die Modifizierung meist hydrolyseempfindliche Stoffe wie Trimethylchlorsilan und Hexamethyldisilazan eingesetzt werden, wird in der Regel zunächst ein Lösungsmittelaustausch durchgeführt. Die wasserhaltige Porenflüssigkeit wird dabei in mehreren Lösungsmittelaustauschschritten durch inerte, organische Lösungsmittel wie Hexan ersetzt, um die Reaktion des Hydrophobierungsmittels (z.B. Trimethylchlorsilan) mit dem Wasser der Porenflüssigkeit zu vermeiden.

Neben der Stabilisierung des Gerüstes im Trocknungsschritt führt die Oberflächenmodifizierung zu einer Hydrophobierung der äußeren und inneren Oberfläche der Aerogele. Für viele Anwendungen ist eine ausreichende Hydrophobie unerlässlich. Insbesondere in der Bauisolierung müssen Dämmstoffe dauerhaft wasserabweisend sein, weshalb hydrophobe Materialien für solche Anwendungen bevorzugt werden.

Die hohe spezifische Oberfläche der Aerogele eröffnet den Einsatz als Trägermaterial und Transportagens in der Chemie beispielsweise für die Katalyse oder in der Medizin. Des Weiteren eignen sich Aerogele durch die spezifische Oberfläche auch zum Einsatz als Absorber- oder Filtermaterialen.

Zu der herausragendsten Eigenschaft von Aerogelen gehört ihre außergewöhnlich geringe thermische Leitfähigkeit. Die hohe Isolierwirkung wird durch den besonderen Aufbau der Aerogele, insbesondere ihre außergewöhnlichen Porenstruktur (Dichten unter 0,2 g/cm$^3$, Mesoporenvolumia über 3 cm$^3$/g und Porendurchmesser unter 25 nm), ermöglicht.

Wärmedämmung (auch Wärmeisolierung genannt) ist ein wichtiger Aspekt zur Verminderung des Energieverbrauchs. Insbesondere in der Bauisolierung gelangen konventionelle, kostengünstige Dammmaterialien wie Polystyrol, Polyurethan und Glaswolle aufgrund ihrer hohen Brennbarkeit und/oder begrenzten Isolierwirkung zunehmend an ihre Grenzen.

Für einen kompetitiven Einsatz der Aerogele ist eine kostengünstige Herstellung essentiell. Daher ist es vorteilhaft, möglichst wenige Prozessschritte durchzuführen und insbesondere besonders auf zeitaufwändige Schritte wie einen mehrstufigen Lösungsmittelaustausch zu verzichten.

Dementsprechend wurde in EP 0 948 395 B1 ein Verfahren zur Herstellung von organisch modifizierten Aerogelen entwickelt, in dem ein Hydrogel direkt oberflächenmodifiziert wird, ohne vorher die wässrige Porenflüssigkeit durch organische Lösungsmittel zu ersetzen. In den Beispielen wird eine Natriumwasserglaslösung oder Siliciumtetrachlorid als $SiO_2$-Quelle verwendet und Hexamethyldisiloxan (HMDSO, $(CH_3)_3Si$-O-$Si(CH_3)_3$), Trimethylchlorsilan (TMCS, $(CH_3)_3SiCl$) oder Trimethylsilanol ($(CH_3)_3SiOH$) zur Modifizierung eingesetzt. Die freien OH-Gruppen des Hydrogels reagieren dabei mit den Silylierungsagenzien zu Sauerstoff -gebundenen Trimethylsilylgruppen (TMS, $(CH_3)_3SiO_{1/2}$). Wird die Silylierung so durchgeführt, dass ein Teil des Wassers in den Poren des Hydrogels mit dem verwendeten Silylierungsmittel (z.B. TMCS) zu dem wasserunlöslichen Hexamethyldisiloxan reagiert, so wird durch das Volumen der gebildeten Verbindung notwendigerweise mindestens ein Teil des Wassers aus den Poren verdrängt. Dies führt während der Silylierung der inneren Oberfläche des Netzwerks zu einem gleichzeitigen, vollständigen oder teilweisen Austausch der Flüssigkeit in den Poren des Hydrogels durch das wasserunlösliche Medium.

Das offenbarte Verfahren hat den Nachteil, dass die Oberflächenmodifizierung (Silylierung) entweder bei hohen Temperaturen von 80-100 °C stattfindet oder eine sehr lange Reaktionszeit von mehreren Tagen erforderlich ist. Nur durch die Verwendung großer Mengen an HCl und/oder Trimethylchlorsilan gelingt hierbei eine schnelle und vollständige Oberflächenmodifizierung. Während der Hydrophobierung wird die Porenflüssigkeit aus dem Gel verdrängt und durch HMDSO ersetzt, wobei die Autoren dieses Patents, F. Schwertfeger und D. Frank, in einer nachfolgenden Publikation mit M. Schmidt im Journal of non-Crystalline Solids (Vol. 225, S. 24-29, 1998) konkretisieren, dass sie für einen vollständigen Austausch der Porenflüssigkeit mindestens 15 mol-% TMCS bezogen auf das Porenwasser, das entspricht 81,5 g TMCS je 100 g Hydrogel (s. Probe 2 in Tab. 1), benötigen, um einen vollständigen Austausch der Porenflüssigkeit und damit Aerogele geringer Dichte (kleiner 140 kg/m$^3$) zu erhalten. Dabei entstehen je 100 g Hydrogel 80 ml HMDSO als Nebenprodukt. Neben den anfallenden Rohstoffkosten entsteht zudem ein Entsorgungsproblem. Die wässrige, zum Teil mit Salzen verunreinigte HCl kann in der Regel nicht recycelt werden, sondern muss über das Abwasser entsorgt werden. Durch den hohen Überschuss an Trimethylchlorsilan entsteht eine große Menge an Hexamethyldisiloxan, das erst durch einen zusätzlichen Prozessschritt wieder in Trimethylchlorsilan überführt werden kann. Auch die bei der Verwendung großer Mengen an TMCS entstehende Reaktionswärme erfordert einen erhöhten prozesstechnischen Aufwand. Deshalb, aber auch um die Menge an Gefahrstoffen zu minimieren und dadurch die Prozesssicherheit zu erhöhen, sollte die Menge an Salzsäure und Trimethylchlorsilan minimiert werden.

Auch die Autoren der Patente CN 101691227 und CN 102897779 führen eine Oberflächenmodifizierung des Lyogels durch, um auf eine überkritische Trocknung verzichten zu können und damit die Produktionskosten zu reduzieren, sowie ein einfaches Verfahren zur Herstellung silikatischer Aerogele zur Verfügung zu stellen. Im Gegensatz zu EP 0 948 395 B1 und der genannten Publikation von Schwertfeger, Frank und Schmidt, werden in CN 101691227 und CN 10897779 durch Cokondensation von Methyltrimethoxysilan (MTMS) oder Methyltriethoxysilan (MTES) mit $SiO_2$-Quellen wie Wasserglas oder TEOS bereits "vormodifizierte" silikatische Gele eingesetzt und nach einem Lösungsmittelaustausch die erhaltenen silikatischen Lyogele im zweiten Schritt mit einer Lösung aus TMCS in Hexan oberflächenmodifiziert. Der Lösungsmittelaustausch und die Verdünnung des Silylierungsmittels in Hexan führen allerdings zu sehr langen Reaktionszeiten, was eine großtechnische Umsetzung erschwert. Der Umgang mit Chlorsilanen wie TMCS hat den Nachteil, dass beispielsweise wegen des geringen Siedepunkts von TMCS (57 °C) bei höheren Temperaturen keine offenen Systeme benutzt werden können. Zudem ist TMCS als leicht entzündlicher, ätzender und toxischer Stoff eingestuft. Eine Substitution von TMCS durch Salzsäure, die bei der Verwendung von HMDSO als Lösungsmittel möglich ist, ist bei der Verwendung von Hexan als Lösungsmittel nicht möglich. Nach der Reaktion müssen Hexan, TMCS, sowie das bei der Reaktion von TMCS mit dem Porenwasser entstehende HMDSO getrennt oder entsorgt werden, was zusätzliche Kosten verursacht.

Aufgabe der Erfindung ist es daher, ein wirtschaftliches Verfahren für die Herstellung von hydrophobierten Aerogelen zur Verfügung zu stellen, das kostengünstig, einfach und sicher in der Handhabung ist und ressourcenschonend arbeitet.

Diese Aufgabe wird gelöst durch die Bereitstellung eines Verfahrens zur Herstellung organisch modifizierter Aerogele, indem ein Sol enthaltend [$SiO_{4/2}$]-Einheiten und [$R_xSiO_{(4-x)/2}$]-Einheiten, hergestellt wird, wobei bereits bei der Herstellung des Sols ein Phasenvermittler zugegeben oder ein Phasenvermittler aus dem Sol gebildet wird, aus dem Sol ein Gel gebildet wird, wobei der Gehalt an Phasenvermittler in der Porenflüssigkeit zwischen 1 und 50 Gew.-% liegt, das erhaltene Gel in Anwesenheit von über 0,1 Gew.-% des Phasenvermittlers in einem Gemisch enthaltend ein Disiloxan der Formel $R_3Si$-O-$SiR_3$ und Initiator oberflächenmodifiziert wird, wobei das Gemisch mindestens 20 Gew.-% des Disiloxans der Formel $R_3Si$-O-$SiR_3$ enthält und wobei der Initiator aus Säure oder Chlorsilan oder Gemischen daraus besteht und die erhaltenen Gele getrocknet werden, wobei X gleich oder verschieden sein kann und 1, 2 oder 3 ist und wobei R gleich oder verschieden sein kann und Wasserstoff oder ein organischer, substituierter oder unsubstituierter Rest ist.

Wie bereits in der Einleitung beschrieben, sind Aerogele hochporöse Festkörper, bei denen die Poren mit Luft gefüllt sind. Dagegen wird unter einem Lyogel ein Gel verstanden, dessen Poren mit Lösungsmittel gefüllt sind. Die Porenflüssigkeit eines Hydrogels besteht zu mindestens 50% aus Wasser.

Zunächst wird ein Sol enthaltend $[SiO_{4/2}]$-Einheiten und $[R_xSiO_{(4-x)/2}]$-Einheiten (wobei x gleich oder verschieden sein kann und 1, 2 oder 3 ist; und R gleich oder verschieden sein kann und R Wasserstoff oder ein organischer, substituierter oder unsubstituierter Rest ist) hergestellt. In der vorliegenden Erfindung bezeichnet ein Sol eine Lösung und/oder kolloidale Dispersion von Molekülen und/oder Partikeln in mindestens einem Lösungsmittel bzw. Dispersionsmedium.

Ein Lösungsmittel (auch Lösemittel oder Solvens) ist ein Stoff, der Gase, Flüssigkeiten oder Feststoffe lösen oder verdünnen kann, ohne dass es dabei zu chemischen Reaktionen zwischen gelöstem Stoff und lösendem Stoff kommt.

Eine Dispersion ist ein heterogenes Gemisch aus mindestens zwei Stoffen, die sich nicht oder kaum ineinander lösen oder chemisch miteinander verbinden. Dabei ist ein oder mehrere Stoffe (disperse Phase) fein verteilt in einem anderen kontinuierlichen Stoff (Dispersionsmedium, Synonym = kontinuierliche Phase). Nach ihrer Teilchengröße bezeichnet man als kolloidal dispers gelöst disperse Phasen mit einer Teilchengröße von typischerweise ca. 1 nm bis 1 $\mu$m.

Die Reste R können gleich oder verschieden sein und unabhängig voneinander, Wasserstoff, ein organischer, linearer, verzweigter, cyclischer, gesättigter oder ungesättigter, aromatischer oder heteroaromatischer Rest, mit oder ohne Substituenten. Das bedeutet, dass die Reste R substituiert oder unsubstituiert sein können. Bevorzugte Substituenten sind -CN, -NCO, -NR$_2$,-COOH, -COOR, -Halogen, -(Meth)acryl, -Epoxy, -SH, -OH, -CONR$_2$,-O-R, -CO-R, -COO-R, -OCO-R, oder -OCOO-R, -S-R, -NR-, -N=R,-N=N-R, oder -P=R. Vorzugsweise werden gesättigte oder ungesättigte Reste mit $C_1$-$C_4$-, besonders bevorzugt $C_1$-$C_4$-Alkyl, Vinyl, insbesondere Methyl oder Ethyl, im speziellen Methyl eingesetzt.

Die $[SiO_{4/2}]$-Einheiten bezeichnen Verbindungen, in denen ein Siliciumatom an vier Sauerstoffatome gebunden ist, die wiederum jeweils ein freies Elektron für eine weitere Bindung aufweisen. Es können über das Sauerstoffatom verbundene Einheiten mit Si-O-Si-Bindungen vorliegen. Die freien Sauerstoffatome sind im einfachsten Fall an Wasserstoff oder Kohlenstoff gebunden bzw. die Verbindungen liegen als Salze bevorzugt Alkalisalze vor.

Als Ausgangsstoff (Vorstufe) zur Bildung von $[SiO_{4/2}]$-Einheiten ($[SiO_{4/2}]$-Ausgangsstoff) können dem Fachmann bekannte, kondensationsfähige tetra- oder höherfunktionelle Silane, Alkoxysilane, Alkylsilicate, Alkalisilicate oder kolloidale Silica-Partikel bzw. -Lösungen verwendet werden.

Es ist bevorzugt, als Ausgangsstoff für $[SiO_{4/2}]$-Einheiten Verbindungen das Typs $Si(OR)_4$, $[SiO_{4/2}]_w[SiO_{3/2}(OR)]_x[SiO_{2/2}(OR)_2]_y[SiO_{1/2}(OR)_3]_z$ (mit w, x, y, z als nicht negative, ganze Zahl), $SiCl_4$, Wassergläser oder kolloidale Silica-Lösungen einzusetzen. Für R gilt jeweils die bereits gegebene Definition. Insbesondere bevorzugt wird Tetraethylorthosilicat (TEOS) oder Natriumwasserglas eingesetzt. Es können auch Gemische oder Hydrolyseprodukte der genannten Ausgangsstoffe, insbesondere deren Hydrolyseprodukte mit Wasser und/oder Alkoholen, eingesetzt werden.

Als Wasserglas werden aus einer Schmelze erstarrte, glasartige, also amorphe, wasserlösliche Natrium-, Kalium- und Lithiumsilicate oder ihre wässrigen Lösungen bezeichnet. Durch Neutralisieren des Salzes und Hydrolyse entstehen aus den kettenartigen Si-O-Si-Verbindungen $[SiO_{4/2}]$-Einheiten.

Insbesondere bevorzugt wird Tetraethylorthosilicat (TEOS) eingesetzt. Die Hydrolyse von TEOS in Wasser kann durch Säuren oder Basen katalysiert werden:

$$C_8H_{20}O_4Si + 4\ H_2O \rightarrow H_4SiO_4 + 4\ C_2H_5OH$$

wobei die gebildete Orthokieselsäure ($H_4SiO_4$) durch Ausbildung von Si-O-Si-Bindungen und Abgabe von Wasser weiter vernetzt, bis stöchiometrisch Siliciumdioxid entsteht:

$$H_4SiO_4 \rightarrow H_2SiO_3 + H_2O$$

$$H_2SiO_3 \rightarrow SiO_2 + H_2O$$

Bevorzugt wird die Hydrolyse von Tetraalkoxysilanen in wässrigen Lösungen mineralischer oder organischer Säuren, insbesondere bevorzugt in wässriger Salzsäurelösung durchgeführt.

Bei den $[R_xSiO_{(4-x)/2}]$-Einheiten (wobei x gleich oder verschieden sein kann und 1, 2 oder 3 ist) sind neben ein, zwei oder drei Sauerstoffatomen ein, zwei oder drei Reste R direkt an das Siliciumatom gebunden. Für R gilt die bereits aufgeführte Definition. Es können entweder in allen $[R_xSiO_{(4-x)/2}]$-Einheiten ein Rest R und drei Sauerstoffatome, d.h. x=1, oder in allen $[R_xSiO_{(4-x)/2}]$-Einheiten zwei Reste R und zwei Sauerstoffatome, d.h. x=2, oder in allen $[R_xSiO_{(4-x)/2}]$-Einheiten drei Reste R und ein Sauerstoffatom, d.h. x=3, an das Siliciumatom gebunden sein. Es können aber auch beliebige Gemische enthaltend $[RSiO_{3/2}]$-Einheiten und/oder $[R_2SiO_{2/2}]$-Einheiten und/oder $[R_3SiO_{1/2}]$-Einheiten vorliegen. Auch hier steht $O_{(4-x)/2}$ (z.B. $O_{3/2}$, $O_{2/2}$ oder $O_{1/2}$) für (4-x) (=3, 2 oder 1) Sauerstoffatome, die jeweils ein freies Elektron für eine weitere Bindung aufweisen.

Durch die Si-R Gruppen der $[R_xSiO_{(4-x)/2}]$-Einheit erhält das Produkt (also das Gel) eine Grundhydrophobierung.

Als Ausgangsstoff (Vorstufe) zur Bildung von $[R_xSiO_{4-x)/2}]$-Einheiten ($[R_xSiO_{(4-x)/2}]$-Ausgangsstoff) können dem Fachmann bekannte, kondensationsfähige bifunktionelle, trifunktionelle oder höher funktionelle Silane, Alkoxysilane oder Siliconate eingesetzt werden. Gegebenenfalls können auch monofunktionelle Silane, Alkoxysilane oder Siliconate eingesetzt werden. Für R gilt die bereits gegebene Definition.

Bevorzugt eingesetzt werden Verbindungen des Typs $RSi(OR)_3$, $RSiCl_3$, $[RSi(OH)_{3-n}(OM)_n]$ (mit n als nicht negative, ganze Zahl zwischen 0 und 3, mit M = Li, Na, K) sowie deren Hydrolyse und/oder Kondensationsprodukte, $[RSiO_{3/2}]_x[RSiO_{2/2}(OR)]_y[RSiO_{1/2}(OR)_2]_z$ (mit x, y, z als nicht negative, ganze Zahl), $R_2Si(OR)_2$, $R_2SiCl_2$, $[R_2Si(OH)_{2-m}(OM)_m]$ (mit m als nicht negative, ganze Zahl zwischen 0 und 2, mit M = Li, Na, K) sowie deren Hydrolyse und/oder Kondensationsprodukte, $[R_2SiO_{2/2}]_y[R_2SiO_{1/2}(OR)]_z$ (mit y, z als nicht negative, ganze Zahl), $R_3SiCl$, $R_3SiOR$, $R_3Si-O-SiR_3$, $R_3Si-NH-SiR_3$, $R_3SiOH$, $R_3SiOM$ (mit M = Li, Na, K). Für R gilt jeweils die bereits gegebene Definition.

Besonders bevorzugt eingesetzt werden Methyltrialkoxysilane, Vinyltrialkoxysilane, Dimethyldialkoxysilane, OH-, OR-, H- oder Cl-terminierte Polydimethylsiloxane, Alkalimethylsiliconate. Es ist insbesondere bevorzugt, als Ausgangsstoff für die $[R_xSiO_{(4-x)/2}]$ -Einheiten Methyltriethoxysilan (MTES), Methyltrimethoxysilan, Kaliummethylsiliconat oder Natriummethylsiliconat einzusetzen. Es können auch Gemische, Hydrolyseprodukte und/oder Kondensationsprodukte der genannten Ausgangsstoffe, insbesondere deren Hydrolyseprodukte mit Wasser und/oder Alkoholen, eingesetzt werden. Insbesondere bevorzugt wird Methyltriethoxysilan (MTES) eingesetzt.

Aus den Ausgangsstoffe für $[SiO_{4/2}]$ - und $[R_xSiO_{(4-x)/2}]$ -Einheiten, sowie gegebenenfalls Zusatz- und Hilfsstoffen wird nach den dem Fachmann bekannten Methoden ein Sol hergestellt. Unter einer Solherstellung versteht man das Mischen von Ausgangsstoffen mit mindestens einem Lösungsmittel bzw. Dispergiermittel. Dabei kann während und/oder nach dem Mischen auch eine Reaktion der Ausgangsstoffe stattfinden.

Sole aus Alkoxysilanen werden beispielsweise durch Hydrolyse unter Freisetzung der entsprechenden Alkohole hergestellt. Die Hydrolyse kann durch Zugabe von Säure und/oder Temperaturerhöhung beschleunigt werden. Sole aus Wassergläsern und/oder Siliconaten werden beispielsweise durch Neutralisation der stark basischen Alkalisilikate bzw. Alkysiliconate hergestellt. Dies kann nach dem Fachmann bekannten Methoden wie z.B. in EP 0 948 395 B beschrieben, durch Neutralisation mit Mineralsäuren und mittels saurer Ionenaustauscherharze erfolgen.

Darüber hinaus können dem Sol Zusatzstoffe wie dem Fachmann bekannte IR-Trübungsmittel zur Reduzierung der Wärmeleitfähigkeit zugesetzt werden. Ebenso können zur Erhöhung der mechanischen Stabilität beschichtete und/oder unbeschichtete Fasern zugesetzt werden. Als Fasermaterialien können anorganische Fasern, wie z. B. Glasfasern oder Mineralfasern, organische Fasern, wie z. B. Polyesterfasern, Aramidfasern, Nylonfasern oder Fasern pflanzlichen Ursprungs, sowie Gemische derselben verwendet werden.

Für die Herstellung von gemischten Solen, also Sole enthaltend $[SiO_{4/2}]$ -Einheiten und $[R_xSiO_{(4-x)/2}]$ -Einheiten werden nach dem Stand der Technik die Ausgangsstoffe gemischt und nach den oben beschriebenen Methoden ein Sol hergestellt. Das gleichzeitige Mischen der Ausgangsstoffe führt zur Cokondensation der verschiedenen Einheiten. Dagegen kann durch eine zeitlich versetzte, bevorzugt spätere Zugabe von mindestens einem Teil der $[R_xSiO_{(4-x)/2}]$ -Ausgangsstoffe in das noch flüssige Sol eine Konzentrationsänderung der $[R_xSiO_{(4-x)/2}]$ -Einheiten in den Primärpartikeln von innen nach außen erzielt werden. Die Konzentration der $[R_xSiO_{(4-x)/2}]$ -Einheiten nimmt in den Primärpartikeln bevorzugt von innen nach außen zu. Durch die spätere Zugabe von einem Teil der $[R_xSiO_{(4-x)/2}]$ -Ausgangsstoffe kann eine Anreicherung der $[R_xSiO_{(4-x)/2}]$ -Einheiten an der Oberfläche der Sol-Partikel und damit ein Gradienten-artiger oder Core-Shell- (Kern-Schale-) artiger Aufbau realisiert und somit eine maximale Grundhydrophobierung bei minimalem Anteil an $[R_xSiO_{(4-x)/2}]$ -Einheiten erreicht werden. Bei einem Gradienten-artigen Aufbau nimmt die Konzentration der $[R_xSiO_{(4-x)/2}]$ -Einheiten von innen nach außen graduell, d.h. allmählich, nicht abrupt zu. Bei einem Core-Shell-artigen Aufbau steigt die Konzentration der $[R_xSiO_{(4-x)/2}]$ -Einheiten von innen nach außen dagegen sprunghaft an. Bevorzugt weist der Kern eine Konzentration an $[R_xSiO_{(4-x)/2}]$ -Einheiten von unter 20 mol-%, besonders bevorzugt von unter 10 mol-% und insbesondere bevorzugt von 0 mol-% auf und die Schale eine Konzentration an $[R_xSiO_{(4-x)/2}]$ -Einheiten von bevorzugt über 80 mol-%, besonders bevorzugt von über 90 mol-% und insbesondere bevorzugt von 100 mol-% auf.

Dabei ist es vorteilhaft, dass die vorgelegten Ausgangsstoffe für $[SiO_{4/2}]$ -Einheiten und für mindestens einen Teil der $[R_xSiO_{(4-x)/2}]$ -Einheiten vor der Zugabe der restlichen oder gegebenenfalls aller Ausgangsstoffe für $[R_xSiO_{(4-x)/2}]$ -Einheiten bereits weitgehend zum Sol umgesetzt wurden. Eine gründliche Umsetzung kann durch eine Temperaturerhöhung und/oder Wartezeit erreicht werden. Eine spätere, zeitlich versetzte Zugabe in das noch flüssige Sol bedeutet im Rahmen dieser Erfindung, dass nach der Zugabe der ersten Ausgangsstoffe bevorzugt 5 Minuten bis 10 Stunden, besonders bevorzugt 30 Minuten bis 5 Stunden, insbesondere bevorzugt 30 Min bis 2 Stunden unter weiterem Rühren inkubiert wird, bevor die restlichen Ausgangsstoffe für $[R_xSiO_{(4-x)/2}]$ zugegeben werden. Dabei wird das flüssige Sol bevorzugt auf eine Temperatur zwischen 5 und 100 °C, besonders bevorzugt zwischen 10 und 80 °C, insbesondere bevorzugt zwischen 15 und 40 °C temperiert.

Eine bevorzugte Ausführungsform des Verfahrens ist es daher, im Schritt der Solherstellung (Schritt i) mindestens 1 Gew.-%, des Weiteren bevorzugt mindestens 25 Gew.-%, besonders bevorzugt mindestens 50 Gew.-% und insbesondere bevorzugt mindestens 80 Gew.-% der Ausgangsstoffe zur Bildung von $[R_xSiO_{(4-x)/2}]$ -Einheiten ($[R_xSiO_{(4-x)/2}]$ -Ausgangsstoffe) erst später zu den bereits vorgelegten Ausgangsstoffen zuzugeben. In einer besonders bevorzugten Ausführungsform wird der gesamte Anteil der [$R_xSiO_{(4-x)/2}$]-Ausgangsstoffe erst später zum Sol gegeben. Es ist also möglich, zunächst ein Sol nur aus den [$SiO_{4/2}$] -Ausgangsstoffen oder ein Sol aus [$SiO_{4/2}$] -Ausgangsstoffen und einen Teil der [$R_xSiO_{(4-x)/2}$]-Ausgangsstoffe herzustellen und den restlichen Teil der [$R_xSiO_{(4-x)/2}$]-Ausgangsstoffe vor/während oder nach dem Start der Gelbildung in das noch flüssige Sol zu geben. Bevorzugt liegt der Anteil der [$R_xSiO_{(4-x)/2}$] -Einheiten (bezogen auf die Summe der [$SiO_{4/2}$] -Einheiten und der [$R_xSiO_{(4-x)/2}$] -Einheiten) im Bereich zwischen 1 und 80 mol-%, besonders bevorzugt zwischen 1 und 60 mol-%, insbesondere bevorzugt zwischen 5 und 50 mol-%. Im Allgemeinen beträgt der Feststoffgehalt, also der Gehalt an [$SiO_{4/2}$] -Einheiten und [$R_xSiO_{(4-x)/2}$] -Einheiten, im Sol zwischen 3 und 30 Gew.-%, bevorzugt zwischen 5 und 20 Gew.-%, besonders bevorzugt zwischen 8 und 15 Gew.-%.

In einer bevorzugten Ausführungsform wird als [$SiO_{4/2}$]-Ausgangsstoff Tetraethoxysilan (TEOS) und als [$R_x$-$SiO_{(4-x)/2}$]-Ausgangsstoff Methyltriethoxysilan (MTES) eingesetzt. Die beiden Ausgangsstoffe werden zunächst gemischt und unter Rühren in Wasser mit Salzsäure als Katalysator überführt. Als Lösungsmittel bzw. Lösungsmittelgemisch für die Solherstellung werden im Allgemeinen Wasser oder homogene Gemische aus Wasser und polaren, organischen Lösungsmitteln, bevorzugt Alkoholen, eingesetzt. Der Wassergehalt ist dabei bevorzugt hoch genug, damit das wasserhaltige Gemisch im Silylierungsmittel eine separate Flüssigphase ausbildet. Wie bereits erwähnt, kann die Zugabe der [$R_xSiO_{(4-x)/2}$] -Vorstufe auch zeitlich versetzt erfolgen, um eine Anreicherung der hydrophoben [$R_xSiO_{(4-x)/2}$] -Einheiten an der Oberfläche der Primärpartikel und schließlich auch an der Oberfläche das Gels zu erreichen, bzw. die Oberfläche der Primärpartikel und des Gels komplett mit [$R_xSiO_{(4-x)/2}$] -Einheiten zu belegen. Die Solbildung kann durch Zugabe von Katalysatoren, bevorzugt Säuren beschleunigt werden. Als Säuren können Mineralsäuren oder organische Säuren eingesetzt werden. Besonders bevorzugt wird Salzsäure eingesetzt. Die Salzsäure dient hierbei als Katalysator für die Hydrolyse der Alkoxygruppen. Die HCl-Konzentration beträgt dabei bevorzugt 10-1000 ppm, bevorzugt 30-300 ppm, insbesondere bevorzugt 100-200 ppm. Zur Beschleunigung der Hydrolyse kann das Reaktionsgemisch erwärmt werden. Dieser Schritt erfolgt bevorzugt bei 40 bis 80 °C, besonders bevorzugt bei 55-65 °C für eine Zeit von bevorzugt 0,1 bis 3 Stunden, besonders bevorzugt 0,5 bis 1 Stunde. Dadurch entsteht ein klares Sol, das gegebenenfalls auch mehrere Stunden bis Tage gelagert werden kann. Bevorzugt wird das Sol aber direkt weiter umgesetzt.

Vergleichbare Sole sind auch auf Basis von Wasserglas, insbesondere Natrium-Wasserglas als [$SiO_{4/2}$] -Vorstufe und Alkalimethylsiliconat, insbesondere Kalium- oder Natriummethylsiliconat, und/oder Methyltrialkoxysilan als [$R_xSiO_{(4-x)/2}$] -Vorstufe über dem Fachmann bekannte Methoden zugänglich (z.B. EP 0 948 395 B oder Beispiel 6). Die Neutralisation der Vorstufen und die Entfernung der dabei anfallenden Salze kann nach allen dem Fachmann bekannten Methoden (Ionenaustauscher, Fällung in Form schwerlöslicher Salze, Auswaschen, usw.) erfolgen.

Im 2. Schritt (ii) des Verfahrens wird aus dem Sol ein Gel gebildet. Die Gelbildung erfolgt nach dem Fachmann bekannten Methoden wie pH-Wert-Änderung und/oder Temperaturerhöhung. Im Anschluss an die Gelbildung kann eine Alterung erfolgen, die ebenfalls nach bekannten Methoden wie pH-Kontrolle und Erhitzen beschleunigt werden kann.

Zum Start der Gelbildung wird das Sol vorzugsweise unter Rühren mit einer Base versetzt und schwach alkalisch gestellt. Für die Gelbildung im Alkyoxysilan-basierten Verfahren wird für die Gelbildung bevorzugt ein pH-Wert von 7 bis 10, besonders bevorzugt zwischen 8,5 und 9,5 eingestellt. Hierzu können im allgemeinen alle dem Fachmann bekannten Basen, wie $NH_4OH$, NaOH, KOH, $Al(OH)_3$, Silicate oder Siliconate eingesetzt werden, bevorzugt wird $NH_4OH$ (Ammoniak), Wasserglas oder Alkalimethylsiliconat eingesetzt. Eine Beschleunigung der Gelbildungszeit kann auch über eine Temperaturerhöhung erreicht werden. Im Allgemeinen wird die Gelbildung bei einer Temperatur zwischen 0 °C und dem Siedepunkt der enthaltenden Lösungsmittel, bevorzugt zwischen 40 und 80 °C, besonders bevorzugt zwischen 50 und 70 °C durchgeführt.

Sole basierend auf Wasserglas als [$SiO_{4/2}$] -Vorstufe werden bevorzugt bei einem pH-Wert zwischen 3 und 10, insbesondere bevorzugt zwischen 4 und 7 und bei einer Temperatur zwischen dem Gefrierpunkt und dem Siedepunkt der enthaltenden Lösungsmittel, bevorzugt zwischen 0 und 60 °C, insbesondere zwischen 0 und 30 °C, im speziellen zwischen 5 und 20 °C in ein Gel überführt. Das erhaltene Gel kann anschließend einer Alterung unterzogen werden. Eine Alterung im Sinne der Erfindung bedeutet, dass das Gel bei einer Temperatur im Bereich von 20 bis 100 °C, vorzugsweise bei 50 bis 70 °C und insbesondere bevorzugt bei 60 °C für 1 Sekunde bis 48 Stunden, vorzugsweise 30 min bis 24 Stunden und insbesondere bevorzugt 30 min bis 3 Stunden und einem pH-Wert von 4-11, vorzugweise 7-10 und insbesondere 8-9 durchgeführt wird.

Im 3. Schritt (iii) des Verfahrens wird das erhaltene Gel in Anwesenheit von über 0,1 Gew.-% eines Phasenvermittlers in einem Gemisch enthaltend ein Disiloxan der Formel $R_3Si-O-SiR_3$ und Initiator oberflächenmodifiziert, wobei das Gemisch mindestens 20 Gew.-% des Disiloxans der Formel $R_3Si-O-SiR_3$ enthält und wobei der Initiator aus Säure oder Chlorsilan oder Gemischen daraus besteht.

Unter einem Phasenvermittler wird in der vorliegenden Anmeldung eine polare Verbindung oder Gemische verschiedener dieser Verbindungen verstanden, die sowohl in der wasserreichen Phase als auch in der organischen Phase eine merkliche Löslichkeit besitzt und somit den Stofftransport zwischen den beiden im Wesentlichen nicht mischbaren Phasen beschleunigt.

Erfindungsgemäß erfolgt die Oberflächenmodifizierung in Anwesenheit von über 0,1 Gew.-% eines Phasenvermittlers. Als Phasenvermittler geeignet sind polare organische Verbindungen oder Gemische davon wie

- Alkohole, insbesondere der chemischen Formel R-OH, wobei R wie oben für Reste R angegeben definiert ist (z.B. Methanol, Ethanol, Isopropanol)
- Ketone, insbesondere der chemischen Formel $R^1R^2C=O$, wobei $R^1$ und $R^2$ gleich oder verschieden und wie oben für Reste R angegeben definiert sind (z.B. Aceton $(CH_3)_2C=O$)
- Ether, insbesondere der chemischen Formel $R^1OR^2$, wobei $R^1$ und $R^2$ gleich oder verschieden und wie oben für Reste R angegeben definiert sind (z.B. Diethylether, Tetrahydrofuran, Dimethoxyethan)
- Ester, insbesondere der chemischen Formel $R^1COOR^2$, wobei $R^1$ und $R^2$ gleich oder verschieden und wie oben für Reste R angegeben definiert sind (z.B. Ethylacetat) und
- grenzflächenaktive Substanzen wie Tenside.

Grenzflächenaktiv nennt man organische Verbindungen, die sich dank ihrer Struktur in der Grenzfläche zwischen zwei Phasen so anordnen, dass sie die Grenzflächenspannung (=Oberflächenspannung) erniedrigen und dadurch z.B. Benetzung ermöglichen. Durch Herabsetzen der Oberflächenspannung fördern sie die Durchmischung von zwei Phasen unter Umständen bis zur Bildung einer Emulsion. Je nach ihrer chemischen Zusammensetzung und Anwendung werden grenzflächenaktive Substanzen als Netzmittel, Detergenzien (Tenside, Seife) oder Emulgatoren bezeichnet.

Die Stoffe enthalten im Allgemeinen je eine Wasser stark anziehende hydrophile ("wasserfreundliche") Gruppe und eine lipophile ("fettfreundliche"), Wassermoleküle nur schwach anziehende (hydrophobe) Kohlenwasserstoffgruppe.

Besonders bevorzugt werden als Phasenvermittler Alkohole verwendet wie beispielsweise Methanol, Ethanol und Isopropanol, insbesondere bevorzugt wird als Phasenvermittler Ethanol verwendet.

Der Phasenvermittler kann sowohl durch eine Reaktion aus den Vorstufen (Hydrolyse) in das Sol bzw. Gel eingebracht als auch direkt zum Sol bzw. Gel oder in die Organosiloxan-Phase gegeben werden (auch Kombinationen sind möglich). Erfindungsgemäß wird der Phasenvermittler bereits bei der Herstellung des Sols zugegeben oder aus diesem gebildet. Das bedeutet, dass bereits nach der Herstellung des Sols (Schritt i) in diesem Phasenvermittler enthalten ist. Der Phasenvermittler kann beispielsweise aus der Hydrolyse von Methyltriethoxysilan (MTES), Tetraethoxysilan (TEOS) oder Dimethyldimethoxysilan (DMDMS) gebildet werden.

Unter einem Silylierungsmittel werden in der vorliegenden Anmeldung Disiloxane der Formel $R_3Si$-O-$SiR_3$, sowie Lösungen dieser in nicht reaktiven, polaren Lösungsmitteln verstanden, wobei im Silylierungsmittel gegebenenfalls Initiatoren wie Chlorsilane, insbesondere Trimethylchlorsilan, Säuren, insbesondere Salzsäure (HCl), sowie aus dem Disiloxan, entstehende Spaltprodukte enthalten sein können. Die Lösungsmittel sind bevorzugt Kohlenwasserstoffe wie Pentan, Hexan, Heptan und Toluol.

Unter Organosiloxanen werden in der vorliegenden Anmeldung lineare, cyclische oder verzweigte Verbindungen des Typs $[R_3SiO_{1/2}]_m[R_2SiO]_n[RSiO_{3/2}]_o[SiO_2]_p$ (mit m, n, o, p als ganze Zahl $\geq$ 0) verstanden, wobei für R die gegebene Definition gilt. Ein lineares Organosiloxan weist beispielsweise die allgemeine Formel $R_3Si$-$[O$-$SiR_2]_n$-$O$-$SiR_3$ auf. Es gilt, dass ein Organosiloxan mindestens eine Si-C-Bindung aufweist, d.h. mindestens ein Rest muss organischer Natur sein.

Erfindungsgemäß können auch Gemische verschiedener Organosiloxane, vorzugsweise flüssiger Organosiloxane eingesetzt werden. Bevorzugt wird als Organosiloxan ein Disiloxan verwendet.

Disiloxane sind chemische Verbindungen mit der Formel $R_3Si$-O-$SiR_3$ oder $[R^1R^2R^3SiO_{1/2}]_2$, wobei wiederum für R oder $R^1$, $R^2$ und $R^3$ die oben gegebene Definition gilt und das Disiloxan mindestens eine Si-C-Bindung aufweist. Es werden symmetrische Disiloxane der Formel $R_3Si$-O-$SiR_3$, insbesondere bevorzugt Hexamethyldisiloxan eingesetzt. Es können auch Mischungen verschiedener Disiloxane, insbesondere Mischungen aus Hexamethyldisiloxan und Divinyltetramethyldisiloxan eingesetzt werden. Bei der Oberflächenmodifizierung reagieren die freien und zugänglichen Silanolgruppen des silikatischen Lyogels mit dem Silylierungsmittel. Aus Si-OH Gruppen entstehen dabei Si-O-$SiR_3$ Gruppen.

Die für die eigentliche Silylierungsreaktion notwendigen Silylierungsmittel können auch aus anderen Substanzen, vorzugsweise anderen Silylierungsmitteln, generiert werden, die z.B. nach dem Fachmann bekannten Reaktionen und Mechanismen (Journal of non-Crystalline Solids (Vol. 225, S. 24-29, 1998), EP 0 948 395 B), z.B. durch Spaltung des Organosiloxans im Sauren, insbesondere durch Spaltung von HMDSO in Salzsäure erfolgen kann. Der Anteil an Disiloxan der Formel $R_3Si$-O-$SiR_3$ im Silylierungsmittel liegt erfindungsgemäß bei mindestens 20 Gew.-%, bevorzugt mindestens 50 Gew.-%, besonders bevorzugt mindestens 90 Gew.-% und insbesondere bevorzugt mindestens 95 Gew.-%. In einer speziell bevorzugten Ausführungsform liegt der Anteil an Organosiloxan im Gemisch bei mindestens 98 Gew.-%, d.h. es wird handelsüblich konzentriertes Disiloxan der Formel $R_3Si$-O-$SiR_3$, besonders bevorzugt Hexamethyldisiloxan verwendet.

Das gebildete Lyogel kann vor, während oder nach einer gegebenenfalls erfolgten Alterung mit Wasser, polaren organischen Lösungsmittels oder Gemischen davon gewaschen werden, um z.B. Elektrolyte zu entfernen. Das Lyogel kann auch ohne Durchführung einer Alterung gewaschen werden. Für den Folgeprozess ist es vorteilhaft, dass die Porenflüssigkeit so viel Wasser enthält, dass die Porenflüssigkeit in dem Silylierungsmittel eine separate Phase bilden

7

kann. Es ist daher besonders bevorzugt, auf einen Lösungsmittelaustausch und/oder Waschschritt komplett zu verzichten, um z.B. die Prozesskosten und -dauer zu minimieren, d.h. dass Schritt iii ohne vorhergehenden Lösungsmittelaustausch stattfindet.

Das gebildete Lyogel kann vor, während oder nach einer gegebenenfalls erfolgten Alterung, bevorzugt während oder nach der gegebenenfalls erfolgten Alterung mit dem Silylierungsmittel gemischt werden. Bevorzugt wird je ml Lyogel mindestens 1 ml, besonders bevorzugt 1-4 ml, insbesondere bevorzugt 1-2 ml Silylierungsmittel eingesetzt.

Es ist bevorzugt das Gel vor, während oder nach der Zugabe des Silylierungsmittels oder vor der Überführung des Lyogels in das Silylierungsmittel, zu zerkleinern. Das hat den Vorteil, dass es zu einer guten Durchmischung, einem großen Anteil von Kontaktflächen des Gels zum Silylierungsmittel und damit schnellen Folgeprozessen, insbesondere einer schnellen Oberflächenmodifizierung kommt. Das Gel wird hierfür durch ein Sieb mit der gewünschten Maschenweite gedrückt. Im Allgemeinen kann die Zerkleinerung aber durch alle dem Fachmann bekannte Methoden, wie Dispergieren, Mahlen, Häckseln, Schneiden, erfolgen.

In einer alternativen bevorzugten Ausführungsform kann auf die Zerkleinerung der fertigen Gelstücke verzichtet werden, indem bereits vor und/oder gleichzeitig mit der Gelbildung in Schritt ii eine Formgebung erfolgt. Besonders bevorzugt erfolgt die Formgebung gleichzeitig mit der Gelbildung.

In einer besonders bevorzugten Ausführungsform wird das flüssige Sol, hergestellt nach einer der beschriebenen Ausführungsformen, in das Silylierungsmittel überführt. Während und/oder nach der Zugabe des Sols erfolgt eine Formgebung der Sol-Tropfen bzw. der entstehenden Gelpartikel durch dem Fachmann bekannte Methoden (beispielsweise Emulgieren, Dispergieren, Versprühen), bevorzugt durch Dispergieren, insbesondere bevorzugt durch Emulgieren. Dispergieren bedeutet, dass das flüssige Sol durch Rühren in einer kontinuierlichen Phase (Dispersionsmedium) verteilt wird und in dieser die Soltröpfchen in Geltröpfchen überführt werden.

Insbesondere bevorzugt ist die kontinuierliche Phase gleichzeitig das Silylierungsmittel. Das bedeutet, dass in dieser bevorzugten Ausführungsform die Formgebung durch Dispergieren des Sols in einer kontinuierlichen Phase erfolgt, wobei die kontinuierliche Phase mindestens 20 Gew.-%, bevorzugt mindestens 50 Gew.-%, besonders bevorzugt mindestens 90 Gew.-% und insbesondere bevorzugt mindestens 95 Gew.-% des Disiloxans enthält. D.h. die kontinuierliche Phase dient gleichzeitig als Reagenz zur Oberflächenmodifizierung.

Die Gelbildung kann dabei vor, während oder nach der Zugabe des Sols in das Silylierungsmittel, nach den bereits beschriebenen Methoden, wie pH-Wert Erhöhung oder/und Temperaturerhöhung, initiiert werden (wie oben unter Schritt ii beschrieben). Falls eine Base zum Start der Gelbildung verwendet wird, kann diese in das Sol und/oder in die kontinuierliche Phase gegeben werden.

Durch diese bevorzugte Ausführungsform lässt sich der Gesamtprozess weiter verbessern, da die Reaktionsprozesse, insbesondere die Oberflächenmodifizierung, aufgrund der kleineren Größe der Gel-Partikel und der guten Durchmischung beschleunigt werden. Des Weiteren können Emulsionen bzw. Dispersionen deutlich besser gefördert werden, die Partikelgrößen können einfach eingestellt werden, und es ist kein zusätzlicher Zerkleinerungsschritt für die Gele notwendig. Dies vereinfacht eine großtechnische Produktion, weil dadurch eine kontinuierliche oder semikontinuierliche Prozessführung ermöglicht wird. In einer speziell bevorzugten Ausführungsform wird als kontinuierliche Phase technisch reines Disiloxan der Formel $R_3Si-O-SiR_3$, bevorzugt Hexamethyldisiloxan, eingesetzt. Dies ist besonders vorteilhaft, da dadurch keine aufwändige Trennung und Aufbereitung von Stoffgemischen erforderlich ist und das Disiloxan der Formel $R_3Si-O-SiR_3$, bevorzugt Hexamethyldisiloxan, nach der Reaktion direkt wiederverwendet werden kann.

Das Sol kann unter Rühren mit einer Base versetzt und innerhalb einer Minute wiederum unter Rühren in das Disiloxan der Formel $R_3Si-O-SiR_3$ überführt werden (s. erfindungsgemäße Beispiele 1-3). Die Gelbildungszeit kann je nach pH-Wert und Temperatur wenige Sekunden bis zu mehreren Stunden dauern. Es ist vorteilhaft, die Gelbildungszeit so einzustellen, dass das Sol bei der Zugabe zum Silylierungsmittel noch flüssig ist, so dass das Sol noch einfach gefördert werden kann und eine kontrollierte Formgebung möglich ist. Es ist vorteilhaft, die Base erst unmittelbar vor dem Dispergieren des Sols durch geeignete, dem Fachmann bekannte Mischapparaturen, insbesondere solche mit kurzen Verweilzeiten wie statische Mischer, zuzugeben. Die Base kann auch bereits in der kontinuierlichen Phase enthalten sein. Durch eine temperaturinduzierte Gelbildung kann auf die Zugabe einer Base auch verzichtet werden. Dabei ist des vorteilhaft die kontinuierliche Phase zu erwärmen. Das Dispergieren des Sols kann in dem Fachmann bekannten Apparaturen (z.B. Rührkessel, Rohrreaktoren) erfolgen. Die Form und Größe der entstehenden Gel-Partikel kann dabei über Parameter wie Rührerdrehzahl, Rührer- und/oder Reaktorgeometrie und dem Verhältnis zwischen Sol und Silylierungsmittel beeinflusst werden. Das Verhältnis kontinuierliche Phase/Sol (Volumen kontinuierliche Phase / Volumen Sol) ist im Allgemeinen größer 1, bevorzugt zwischen 1 und 10, besonders bevorzugt zwischen 1 und 4, insbesondere bevorzugt zwischen 1 und 2. Bevorzugte Bedingungen für die Gelbildung und Alterung wurden bereits in der vorangehenden, bevorzugten Ausführungsform beschrieben (wie oben unter Schritt ii beschrieben).

Vom gebildeten Lyogel kann vor, während oder nach einer gegebenenfalls erfolgten Alterung ein Teil der kontinuierlichen Phase abgetrennt werden (z.B. durch Filtrieren, Dekantieren, Zentrifugieren, Verdampfen), um z.B. die Konzentration an Gelpartikeln in der Dispersion zu erhöhen und somit z.B. Raum-Zeit-Ausbeuten in den Folgeschritten zu erhöhen.

Das gebildete Lyogel kann vor, während oder nach einer gegebenenfalls erfolgten Alterung mit Wasser, polaren organischen Lösungsmitteln oder Gemischen davon gewaschen werden, um z.B. Elektrolyte zu entfernen.

Die Gele hergestellt nach den beschriebenen Ausführungsformen werden oberflächenmodifiziert, wobei die Oberflächenmodifizierung nach einer gegebenenfalls durchgeführten Alterung erfolgt. Unter den Reaktionsbedingungen, die bei der Gelbildung und Alterung herrschen, findet in dem Silylierungsmittel im Allgemeinen keine oder eine nur sehr langsame Oberflächenmodifizierung statt, da die Disiloxane unter diesen Bedingungen nicht bzw. nur sehr langsam silylieren. Disiloxane können durch Zugabe von Säuren oder Säurelieferanten wie Chlorsilane gezielt initiiert werden. Der Begriff Initiator im Sinne dieser Erfindung ist also eine Säure oder ein Chlorsilan oder Gemische daraus, der die Oberflächenmodifizierung beschleunigt. Das ermöglicht eine bessere Prozesssteuerung. Dies ist ein wesentlicher Vorteil des erfindungsgemäßen Verfahrens gegenüber dem im Stand der Technik meist genutzten Gemisch aus Kohlenwasserstoffen und Trimethylchlorsilan. Erfindungsgemäß wird die Oberflächenmodifizierung durch Zugabe von Säuren und/oder Chlorsilanen durch die bereits beschriebenen, dem Fachmann bekannten Reaktionen und Mechanismen, initiiert. Besonders bevorzugt wird Salzsäure oder Trimethylchlorsilan (TMSC) oder deren Gemische als Initiator eingesetzt. Insbesondere bevorzugt wird konzentrierte Salzsäure verwendet. Das TMCS wirkt dabei als Silylierungsmittel, reagiert mit den Silanol-Gruppen der Geloberfläche und führt dadurch zu einer starken Hydrophobierung des Gerüsts. Bei der Verwendung von Salzsäure als Initiator muss sich das aktive Silylierungsmittel zunächst aus dem Disiloxan und Salzsäure bilden (z.B. durch Spaltung von Disiloxan), was in Beispiel 5 b und den Vergleichsbeispielen 1 b und 2 b zu einem merklich verzögerten Start der Oberflächenmodifizierung führt. Durch intensive Vermischung kann diese allerdings verkürzt werden. Es ist daher von Vorteil, die Oberflächenmodifizierung in Emulsion durchzuführen. In einer Nebenreaktion kann das aktive Silylierungmittel auch mit dem Wasser der Porenflüssigkeit zu dem entsprechenden Disiloxan, reagieren. Daher ist es vorteilhaft, die Oberflächenmodifizierung in Disiloxanen bzw. in einer möglichst konzentrierten Disiloxan-Lösung durchzuführen, da dadurch eine ausreichend hohe Konzentration an aktivem Silylierungsmittel, durch die bereits beschriebene Rückreaktion mit Säure, erhalten bleibt. Darüber hinaus wird durch die Vermeidung eines weiteren Lösungsmittels, wie Hexan, ein einfaches und damit wirtschaftliches Stoffrecycling ermöglicht, da lediglich die wässrige Phase und die Disiloxan-Phase getrennt werden müssen. Dies kann nach dem Fachmann bekannten Methoden zur Trennung von organischen und wässrigen Phasen erfolgen (z.B. Absetzbecken, Zentrifuge, Dekanter, Destillation,...). Es ist daher eine besonders bevorzugte Ausführungsform, als Silylierungsmittel ein Gemisch aus Disiloxan und Initiator einzusetzen und auf die Zugabe eines weiteren Lösungsmittels zu verzichten.

Während der Hydrophobierung wird die polare, wasserhaltige Porenflüssigkeit aus dem Gel verdrängt und durch das Disiloxan bzw. durch die Disiloxan-reiche organische Phase, bevorzugt durch HMDSO ersetzt. Da die wässrige Porenflüssigkeit mit der Disiloxan-reichen Phase im Wesentlichen nicht mischbar ist, bildet sich während der Oberflächenmodifizierung eine zweite, wässrige, flüssige Phase. Da die wässrige Phase bevorzugt eine höhere Dichte als die Disiloxan-reiche Phase besitzt und die Gelpartikel bevorzugt in der organischen Phase enthalten sind, ist ermöglicht das erfindungsgemäße Verfahren eine einfache und ressourcenschonende Stofftrennung. Dies ist insbesondere für eine großtechnische Umsetzung von besonderer Bedeutung. Über die Bestimmung der Menge an verdrängter Porenflüssigkeit kann zudem der Reaktionsverlauf verfolgt werden. In Beispiel 5 und den Vergleichsbeispielen 1-4 wird das Volumen der verdrängten Porenflüssigkeit an einer Skala abgelesen, um zu Aussagen über die Geschwindigkeit der Silylierungsreaktion zu gelangen.

Da polare Flüssigkeiten, insbesondere Wasser, in reinen $[SiO_{4/2}]$ -Gelen aufgrund der hohen Hydrophilie und den geringen Porendurchmessern des Netzwerkes sehr stark gebunden sind und die Verträglichkeit bzw. Mischbarkeit von Wasser und HMDSO sehr gering ist, ist das bereits erwähnte Verfahren von Schwertfeger (EP 0 948 395 B1) auf hohe Prozesstemperaturen und/oder hohe Mengen an HCl und/oder TMCS angewiesen. Überraschenderweise ist es durch den Einbau von $[R_xSiO_{(4-x)/2}]$ -Einheiten in Kombination mit dem Einsatz von Phasenvermittlern gelungen, die Oberflächenmodifizierung von Lyogelen in Organosiloxan bzw. Organosiloxanlösungen auch bei niedrigeren Temperaturen und mit deutlich geringeren Mengen an Initiatoren wie HCl und TMCS in prozesstechnisch sinnvollen Zeiten von wenigen Stunden durchzuführen.

Erfindungsgemäß beträgt die Zeit für den Prozess der Oberflächenmodifizierung und Lösungsmittelverdrängung bevorzugt weniger als 12 Stunden, weiter bevorzugt zwischen 15 Minuten und 3 Stunden, insbesondere bevorzugt zwischen 15 Minuten und 2 Stunden und speziell bevorzugt zwischen 15 und 60 Minuten. Die Silylierungsreaktion kann unter- oder oberhalb der Siedepunkte der beteiligten Stoffe durchgeführt werden. Gegebenenfalls kann die Reaktion auch unter Überdruck durchgeführt werden, um z.B. höhere Reaktionstemperaturen erzielen zu können. Bevorzugt wird die Oberflächenmodifizierung zwischen Raumtemperatur und dem Siedepunkt des Silylierungsmittels bzw. der Porenflüssigkeit durchgeführt. Die Reaktion wird bevorzugt zwischen 50 und 90 °C, besonders bevorzugt zwischen 60 und 80 °C durchgeführt. Der Phasenvermittler kann im Sol und/oder dem Silylierungsmittel enthalten sein. Der Phasenvermittler kann vor der Oberflächenmodifizierung dem Silylierungsmittel und/oder dem Gel und/oder dem Sol zugegeben werden. Bevorzugt ist der Phasenvermittler bereits im Sol enthalten. Der Phasenvermittler kann dabei vor, während oder nach der Solherstellung dem Sol zugegeben werden. Besonders bevorzugt wird der Phasenvermittler aus der $[SiO_{4/2}]$-Vorstufe und/oder der $[R_xSiO_{(4-x)/2}]$ -Vorstufe während der Solherstellung gebildet. Besonders bevorzugt wird als Phasenvermittler Ethanol und/oder Methanol eingesetzt, das aus der Hydrolyse der Vorstufen gebildet wird. Der Gehalt an Phasenvermittler in der Porenflüssigkeit liegt erfindungsgemäß zwischen 1 und 50 Gew.-%, insbesondere bevorzugt zwischen 5 und 30 Gew.-%.

Um die Prozesskosten zu minimieren, ist es vorteilhaft den Einsatz an Säure und/oder Chlorsilan zu minimieren. Bevorzugt werden höchstens 20 g, besonders bevorzugt höchstens 10 g und insbesondere bevorzugt höchstens 5 g Initiator pro 100 g Gel eingesetzt. Um eine gute Durchmischung und damit einen schnellen Stofftransport zu ermöglichen, ist es vorteilhaft, während der Oberflächenmodifizierung zu rühren. Es ist daher eine besonders bevorzugte Ausführungsform die Oberflächenmodifizierung in einer Dispersion durchzuführen (Beispiel 1-3).

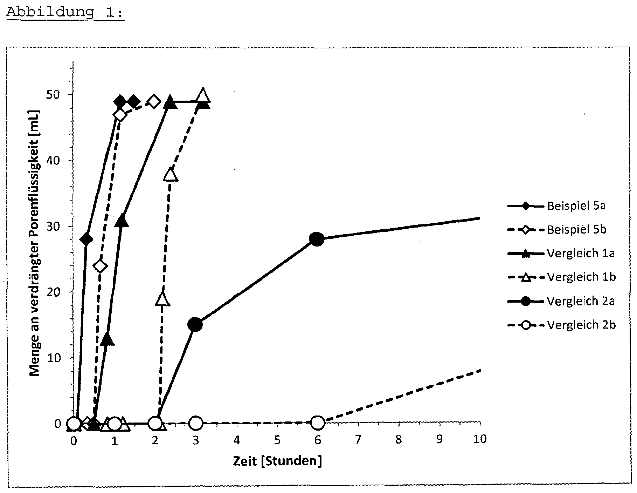

In den Beispielen (Beispiel 5a verglichen mit 5b, Vergleichsbeispiel 1a verglichen mit 1b und 2a mit 2b, s. Abb. 1) konnte gezeigt werden, dass die Hydrophobierung sowohl durch TMCS, als auch durch HCl gestartet werden kann. Es hat sich gezeigt, dass durch die erfindungsgemäße Verwendung von $[R_xSiO_{(4-x)/2}]$ -Einheiten bei der Herstellung des Sols die Verdrängung der wässrigen Porenflüssigkeit im Gel beschleunigt wird (Beispiel 5a bzw. 5b verglichen mit 1a bzw. 1b und 2a bzw. 2b, s. Abb. 1). Einen ersten Hinweis auf diesen Effekt lieferte die Publikation von Z. Shao, F. Luo, X. Cheng und Y. Zhang (Material Chemistry and Physics 141, S. 570-575, 2013). Die Autoren beschreiben, dass der Lösungsmittelaustausch des Porenwassers durch Ethanol aufgrund der Hydrophobierung des Netzwerkes mit MTES verbessert wird. Dennoch ist die Oberflächenmodifizierung mit 48 Stunden, aufgrund der Verwendung eines nichterfindungsgemäßen Silylierungsmittels (Gemisches aus TMCS, Ethanol und Hexan), deutlich zu langsam, um daraus ein wirtschaftliches Verfahren zu entwickeln.

Überraschenderweise wird dieser Effekt deutlich verstärkt, d.h. die Reaktionsgeschwindigkeit deutlich erhöht, wenn gleichzeitig Ethanol als Phasenvermittler und Organosiloxan als Silylierungsmittel eingesetzt wird. Durch die erfindungsgemäße Verwendung der $[R_xSiO_{(4-x)/2}]$ -Einheiten und die Anwesenheit eines Phasenvermittlers wird das Verfahren zur Herstellung des organisch modifizierten Aerogels deutlich beschleunigt. Vergleichsbeispiel 4 zeigt, dass das TMCS-Hexan-Gemisch unter der Verwendung der erfindungsgemäß niedrigen Menge an TMCS und den erfindungsgemäß niedrigen Reaktionstemperaturen selbst nach 24 Stunden keine merkliche Reaktion lieferte. Ohne Grundhydrophobierung (d.h. ohne Zugabe von $[R_xSiO_{(4-x)/2}]$ -Einheiten) und ohne Phasenvermittlung (in Abwesenheit eines Phasenvermittlers) fand mit einem erfindungsgemäßen Silylierungsmittel keine Reaktion statt (Vergleichsbeispiel 3). Durch die erfindungsgemäße Durchführung (Beispiel 5) konnte die Reaktionsgeschwindigkeit im Vergleich zu einer Ausführung ohne Grundhydrophobierung mindestens um den Faktor 2 (Beispiel 5a verglichen mit Vergleichsbeispiel 1a) gesteigert werden. Im Vergleich einer Oberflächenmodifizierung ohne Anwesenheit eines Phasenvermittlers konnte die Geschwindigkeit mindestens um den Faktor 18 beschleunigt werden (Beispiel 5a: 28 ml in 0,33 h verglichen mit Vergleichsbeispiel 2a: 28 ml in 6 h).

Aufgrund der niedrigen Feststoffgehalte im Sol sind bei der Herstellung von Aerogelen im Allgemeinen sehr hohe Stoffvolumen umzusetzen. Die Minimierung der Raum-Zeit-Ausbeute ist bei der Entwicklung eines wirtschaftlichen Verfahrens daher neben der Minimierung der TMCS-Menge entscheidend. So kann durch eine Beschleunigung der Oberflächenmodifizierung um den Faktor 2 der Durchsatz um 100 Prozent gesteigert werden, was zu einer signifikanten Prozesskostenreduktion führt.

Es ist vorteilhaft, die Gelpartikel nach der Oberflächenmodifizierung von der wässrigen Phase und dem überschüssigen Silylierungsmittel abzutrennen. In den erfindungsgemäßen Beispielen wird dies mittels Filtration durchgeführt. Die Stofftrennung kann über alle dem Fachmann bekannten Methoden zur Fest-Flüssig- und/oder Flüssig-Flüssig-Trennung durchgeführt werden (z.B. Dekanter, Absitzbecken, Zentrifugen, Wasserabscheider, Destillation,...). Das Gel bzw. das Reaktionsgemisch kann vor, während oder nach der Abtrennung mit Lösungsmitteln gewaschen und/oder extrahiert werden. Es ist besonders vorteilhaft, das Reaktionsgemisch mit Wasser zu waschen, um die Elektrolyte zu entfernen.

Im nächsten Schritt des erfindungsgemäßen Verfahrens wird das oberflächenmodifizierte und gegebenenfalls gewaschene Gel getrocknet. Im erfindungsgemäßen Beispiel wird das Gel in einem Vakuumtrockenschrank bei 0,01 bar und 80 °C bis zur Gewichtskonstanz getrocknet. Im Allgemeinen kann die Trocknung sowohl im überkritischen Bereich als auch im unterkritischen Bereich erfolgen. Bevorzugt findet eine Trocknung unterhalb des kritischen Punktes statt, vorzugsweise bei Temperaturen von -30 bis 200 °C, besonders bevorzugt 0 bis 150 °C, sowie bei Drücken vorzugsweise von 0,001 bis 20 bar, besonders bevorzugt 0,01 bis 5 bar, insbesondere 0,01 bis 2 bar. Die Trocknung kann dabei durch Strahlungs-, Konvektions- und/oder Kontakttrocknung erfolgen. Die Trocknung wird vorzugsweise so lange durchgeführt, bis das Gel einen Lösungsmittel-Restgehalt von weniger als 0,1 Gew.-% aufweist.

Vor, während oder nach der Trocknung, bevorzugt während oder nach der Trocknung, kann gegebenenfalls eine Agglomeration der Gele zu größeren Partikeln erfolgen. Dies kann beispielsweise nach bekannten Verfahren erfolgen (s. z.B. US 6,481,649 B1 und US 6,620,355 B1).

Die vorliegende Erfindung stellt daher ein kostengünstiges, einfaches, in der Handhabung sicheres und ressourcenschonendes und somit wirtschaftliches Verfahren zur Herstellung organisch modifizierter Aerogele zur Verfügung. Das erfindungsgemäße Verfahren zeichnet sich besonders durch die Kombination der Vorteile eines relativ schnellen Reaktionsverlaufs, Verwendung relativ geringer Mengen Initiator bei relativ milden Temperaturbedingungen aus.

Die besonders bevorzugte Ausführungsform (Gelherstellung und Oberflächenmodifizierung in Dispersion) stellt ein Verfahren zur Verfügung, das aufgrund eines besonders effizienten Stoffrecyclings, schnellem Stofftransport und schnellen Reaktionsschritten die Realisierung eines kontinuierlichen, großtechnischen Prozesses ermöglicht. Die einzelnen Vorteile wurden oben bereits detailliert diskutiert.

Die nach dem erfindungsgemäßen Verfahren herstellbaren Aerogele sind hydrophob. Der molare Anteil an der $[R_xSiO_{(4-x)/2}]$ -Einheiten im Aerogel liegt bevorzugt im Bereich von 1 bis 99 %, besonders bevorzugt im Bereich von 5 bis 60 %, insbesondere bevorzugt im Bereich von 5 bis 50 %. Die Dichte der Aerogele liegt bevorzugt im Bereich von 0,05 bis 0,3 g/cm$^3$, besonders bevorzugt im Bereich von 0,08 bis 0,2 g/cm$^3$, insbesondere bevorzugt im Bereich von 0,09 und 0,15 g/cm$^3$. Die Oberfläche der Aerogele, ermittelt mit der BET-Methode, liegt bevorzugt im Bereich zwischen 300 und 1000 m$^2$/g, besonders bevorzugt zwischen 500 und 900 m$^2$/g, insbesondere zwischen 600 und 800 m$^2$/g. Die erfindungsgemäß hergestellten Aerogele zeichnen sich durch ein BJH-Porenvolumen von bevorzugt mindestens 2,0 cm$^3$/g, besonders bevorzugt mindestens 3 cm$^3$/g, insbesondere bevorzugt mindestens 4,0 cm$^3$/g aus. Die erfindungsgemäß hergestellten Aerogele besitzen eine geringe Wärmeleitfähigkeit. Diese liegt bei den erfindungsgemäß hergestellten Aerogelen, bestimmt bei einer Messtemperatur von 20 °C, vorzugsweise bei weniger als 0,02 W/mK, besonders bevorzugt bei weniger als 0,015 W/mK und insbesondere bei weniger als 0,01 W/mK.

Die erfindungsgemäß herstellbaren organisch modifizierten Aerogele werden daher bevorzugt für Isolierungsanwendungen eingesetzt, besonders bevorzugt in der thermischen und/oder akustischen Isolierung und insbesondere bevorzugt als Wärmedämmstoff.

Die Erfindung wird im Folgenden anhand von Ausführungsbeispielen näher beschrieben, ohne dadurch beschränkt zu werden.

**Analysemethoden:**

Bestimmung der Schüttdichte

Die Schüttdichte wurde in Anlehnung an DIN 53468 ermittelt, indem das Aerogelpulver ohne weitere Verdichtung in ein zylindrisches Gefäß bekannten Volumens (50 cm$^3$) geschüttet und anschließend das Gewicht des Aerogelpulvers über Wiegen ermittelt wurde.

Bestimmung der Dichte

Die Dichte der Aerogelstücke wurde mittels Pyknometrie bestimmt. Hierzu wurden die Aerogelstücke auf einer Analysenwaage gewogen ($m_1$) und zur Volumenbestimmung die Wasserverdrängung in einem 25 ml Pyknometer (Glaspyknometer nach Gay-Lussac nach DIN ISO 3507 der Firma Blaubrand) bei Raumtemperatur gemessen. Hierfür wurden folgende Massen auf einer Analysenwaage bestimmt:

$m_2$: Masse des Pyknometers gefüllt mit destilliertem Wasser
$m_3$: Masse des Pyknometers gefüllt mit dem Aerogelstück und destilliertem Wasser

Das Volumen des Aerogelstücks ($V_1$) entspricht dem Volumen des verdrängten Wassers ($V_2$). Das Volumen und die Dichte des Aerogelstücks wurden nach folgenden Formeln berechnet:

$$V_1 = V_2 = \rho_w * (m_2 - (m_3 - m_1))$$

$$\rho_{Aerogel} = m_1 / V_1$$

wobei $\rho_w$ die Dichte von Wasser bei Raumtemperatur (0,998 g/cm$^3$) angibt.

Bei dem Befüllen des Pyknometers mit dem Aerogelstück und dem Wasser wurde darauf geachtet, dass keine Luftblasen eingeschlossen werden. Aufgrund der hohen Hydrophobie der Aerogelproben ist ein Eindringen von Wasser in die Poren der Proben ausgeschlossen. Zur Kontrolle wurde das Gewicht der Aerogelstücke nach der Messung durch erneutes Wiegen bestätigt.

Bestimmung der BET-Oberfläche

Die spezifische Oberfläche der Aerogele wurde bestimmt nach der BET-Methode entsprechend DIN 9277/66131 und 9277/66132).

Bestimmung des BJH-Porenvolumens und des mittleren Porendurchmessers

Die Porenanalyse wurde nach der Methode von Barett Joyner und Halenda (BJH, 1951) entsprechend DIN 66134 durchgeführt. Für die Auswertung wurden die Daten der Desorptionsisotherme genutzt.

Bestimmung der Ausbeute

Zur Bestimmung der Ausbeute wurden die Gelpartikel bis zur Gewichtskonstanz getrocknet und anschließend bei Raumtemperatur gewogen.

Bestimmung der thermischen Leitfähigkeit

Die thermische Leitfähigkeit wurde mit einem THB Transient Hot Bridge Analyzer (THB-100) der Firma Linseis unter Verwendung eines THB Hot Point Sensors (3x3 mm Kapton) bei Raumtemperatur bestimmt (Messzeit 100 Sekunden, Stromstärke: 5 mA, Heizleistung: 2 mW). Die Größe der vermessenen Aerogelstücke betrug ca. 10x10 mm.

Bestimmung des pH-Werts

Der pH-Wert wurde mit einem pH-Meter der Firma Mettler Toledo Seven Multi; Elektrode: In Lab Science ermittelt.

**Beispiele**

Bezugsquellen gültig für alle Beispiele:

Tetraethylorthosilikat (WACKER® TES28 der Wacker Chemie AG), Methyltriethoxysilan (SEMICOSIL® M3E der Wacker Chemie AG), Dimethyldimethoxysilan (Sigma-Aldrich, Grade: 95%), Wasserglas (Sigma-Aldrich: $SiO_2$-Gehalt: 26,5 Gew.-%, $Na_2O$-Gehalt: 10,6 Gew.-%), Kaliummethylsiliconat (SILRES® BS 16 der Wacker Chemie AG), Hexamethyldisiloxan (WACKER® AK 0,65 SILICONOEL der Wacker Chemie AG), Trimethylchlorsilan (SILAN M3 der Wacker Chemie AG) .
Alle weiteren Laborchemikalien, soweit nicht gesondert erwähnt, wurden von Sigma-Aldrich bezogen.
Für die Aerogel Herstellung in Emulsion (Beispiele 1-3) wurde, falls nicht anders beschrieben, in den Rührschritten ein KPG-Rührer mit einer Drehzahl von 400 Umdrehungen pro Minute (UpM) eingesetzt.

**Beispiel 1:**

In einem Rundkolben wurden 675 ml Wasser und 3,3 ml einer 1 M Salzsäure vorgelegt und auf 60 °C erhitzt. Unter intensivem Rühren wurde eine Mischung aus 156 g TEOS und 134 g MTES zugegeben und für 2 Stunden bei 60 °C gerührt. Das molare Verhältnis TEOS:MTES betrug 1:1.
In einem zweiten Kolben wurden 2000 ml Hexamethyldisiloxan intensiv gerührt.
Das warme Sol wurde unter intensivem Rühren mit 40 ml einer 0,25 M Ammoniaklösung versetzt und innerhalb einer Minute in den zweiten Rundkolben mit dem HMDSO überführt. Das Reaktionsgemisch wurde für 1 Stunde bei Raumtemperatur und anschließend 2 Stunden bei 60 °C gerührt. Während dieser Zeit fand die Gelbildung und Alterung statt. Anschließend wurden unter Rühren 100 g konzentrierte Salzsäure zugegeben und das Reaktionsgemisch für 60 Minuten bei 60 °C gerührt, wobei sich eine wässrige Phase gebildet hat.
Die Gelpartikel wurden durch Filtration mittels eines Büchner-Trichters (Whatman® Filter, 125 mm, Grade 40) abgetrennt. Die Gelpartikel wurden anschließend in einem Vakuumtrockenschrank getrocknet (10 mbar, 80 °C). Die folgenden Werte wurden wie in den Analysemethoden beschrieben, bestimmt.

Schüttdichte: 0,11 g/cm$^3$
BET: 750 m$^2$/g
BJH-Porenvolumen: 4,0 cm$^3$/g
Mittlerer Porendurchmesser: 22 nm
Ausbeute: 117,3 g

**Beispiel 2:**

In einem Rundkolben wurden 675 ml Wasser und 3,3 ml einer 1 M Salzsäure vorgelegt und auf 60 °C erhitzt.

Unter intensivem Rühren wurde eine Mischung aus 226 g TEOS und 64 g MTES zugegeben und für 2 Stunden bei 60 °C gerührt. Das molare Verhältnis TEOS:MTES betrug 3:1.

In einem zweiten Kolben wurden 2000 ml Hexamethyldisiloxan intensiv gerührt.

Das warme Sol wurde unter intensivem Rühren mit 36 ml einer 0,25 M Ammoniaklösung versetzt und innerhalb einer Minute in den zweiten Rundkolben mit dem HMDSO überführt. Das Reaktionsgemisch wurde für 1 Stunde bei Raumtemperatur und anschließend 2 Stunden bei 60 °C gerührt. Während dieser Zeit fand die Gelbildung und Alterung statt. Anschließend wurden unter Rühren 100 g konzentrierte Salzsäure zugegeben und das Reaktionsgemisch für weitere 2 Stunden bei 60 °C gerührt, wobei sich durch die Hydrophobierung und dem damit verbundenen Lösemittelaustausch eine wässrige Phase gebildet hat.

Die Gelpartikel wurden durch Filtration mittels eines Büchner-Trichters (Whatman® Filter, 125 mm, Grade 40) abgetrennt.

Die Gelpartikel wurden anschließend in einem Vakuumtrockenschrank getrocknet (10 mbar, 80 °C). Die folgenden Werte wurden wie in den Analysemethoden beschrieben, bestimmt.

Schüttdichte: 0,14 g/cm$^3$
BET: 850 m$^2$/g
BJH-Porenvolumen: 4,2 cm$^3$/g
Mittlerer Porendurchmesser: 15 nm
Ausbeute: 108,7 g

**Beispiel 3:**

In einem Rundkolben wurden 650 ml Wasser und 3 ml einer 1 M Salzsäure vorgelegt und auf 60 °C erhitzt. Unter intensivem Rühren wurde eine Mischung aus 271 g TEOS und 26 g MTES zugegeben und für 1,5 Stunden bei 60 °C gerührt. Das molare Verhältnis TEOS:MTES betrug 9:1.

In einem zweiten Kolben wurden 1500 ml Hexamethyldisiloxan auf 60 °C erhitzt und intensiv gerührt.

Das warme Sol wurde unter intensivem Rühren mit 37 ml einer 0,25 M Ammoniaklösung versetzt und innerhalb einer Minute in den zweiten Rundkolben mit dem HMDSO überführt. Das Reaktionsgemisch wurde für 3 Stunden bei 60 °C gerührt. Anschließend wurden unter Rühren 100 g konzentrierte Salzsäure zugegeben und das Reaktionsgemisch für weitere 2 Stunden bei 60 °C gerührt, wobei sich durch die Hydrophobierung und dem damit verbundenen Lösemittelaustausch eine wässrige Phase gebildet hat.

Die Gelpartikel wurden durch Filtration mittels eines Büchner-Trichters (Whatman® Filter, 125 mm, Grade 40) abgetrennt. Die Gelpartikel wurden anschließend in einem Vakuumtrockenschrank getrocknet (10 mbar, 80 °C). Die folgenden Werte wurden wie in den Analysemethoden beschrieben, bestimmt.

Schüttdichte: 0,12 g/cm$^3$
BET: 840 m$^2$/g
BJH-Porenvolumen: 4.2 cm$^3$/g
Mittlerer Porendurchmesser: 16 nm
Ausbeute: 113 g

**Beispiel 4:**

In einem Rundkolben wurden 449 g Wasser und 0,7 ml einer wässrigen Salzsäure (1 M) vorgelegt und auf 60 °C erhitzt. Unter intensivem Rühren wurde eine Mischung aus 104 g TEOS und 89 g MTES zugegeben und für 2 Stunden bei 60 °C gerührt. Das molare Verhältnis TEOS:MTES betrug 1:1.

Nach dem Abkühlen auf Raumtemperatur wurde das Sol in einem Becherglas mit 25 ml einer 0,25 molaren Ammoniak-Lösung versetzt. Für die Alterung wurde das erhaltene Gel in einem geschlossenen Gefäß für 3 Stunden bei 60 °C in einem Trockenschrank inkubiert. Anschließend wurde das Gel in Stücke von ca. 10 mm zerteilt. 50 g der Gelstücke wurden mit 100 ml HMDSO überschichtet und bei 50 °C mit 5,0 g TMCS versetzt und in einer verschlossenen Schraubflasche für 2 Stunden bei 50 °C in einem Trockenschrank inkubiert. Anschließend wurden die Gelstücke durch Filtration mittels eines Büchner-Trichters (Whatman® Filter, 125 mm, Grade 40) abgetrennt und in einem Vakuumtrockenschrank (80 °C und 10 mbar) bis zur Gewichtskonstanz getrocknet. Versuch 1 und Versuch 2 stellen 2 unabhängige Durchführungen von Beispiel 4 dar. Die folgenden Werte wurden wie in den Analysemethoden beschrieben, bestimmt.

**Versuch 1:**

Dichte: 0,12 g/cm$^3$
BET-Oberfläche: 795 m$^2$/g
BJH-Porenvolumen: 4,23 cm$^3$/g
Mittlerer Porendurchmesser: 22,4 nm
Thermische Leitfähigkeit: 0,011 W/m*K

**Versuch 2:**

Dichte: 0,11 g/cm$^3$
BET-Oberfläche: 804 m$^2$/g
BJH-Porenvolumen: 3,97 cm$^3$/g
Mittlerer Porendurchmesser: 18,2 nm
Thermische Leitfähigkeit: 0,01 W/m*K

Die Wärmeleitfähigkeit hat die Einheit Watt je Meter und Kelvin (W/m*K).

**Beispiel 5:** Ermittlung der Hydrophobierungs- bzw. Lösemittelaustauschgeschwindigkeiten bei Gelen mit Grundhydrophobierung und Anwesenheit eines Phasenvermittlers

In einem Rundkolben wurden 449 g Wasser und 0,7 ml einer wässrigen Salzsäure (1 M) vorgelegt und auf 60 °C erhitzt. Unter intensivem Rühren wurde eine Mischung aus 104 g TEOS und 89 g MTES zugegeben und für 2 Stunden bei 60 °C gerührt. Nach dem Abkühlen auf Raumtemperatur wurde das Sol in einem Becherglas mit 25 ml einer 0,25 molaren Ammoniak-Lösung versetzt. Anschließend wurde das erhaltene Gel gealtert, d.h. in einem geschlossenen Gefäß für 3 Stunden bei 60 °C in einem Trockenschrank inkubiert. Danach wurde das Gel durch ein Sieb mit einer Maschenweite von 5 mm gedrückt, um Stücke kleiner 5 mm zu erhalten. Die Gelstücke wurden anschließend entsprechend der Varianten a), b) und c) umgesetzt.

a) In einer verschlossenen Schraubflasche wurden 50 g Gel und 100 ml HMDSO bei 50 °C mit 5,0 g Trimethylchlorsilan versetzt, geschüttelt und die Menge der aus dem Gel verdrängten wässrigen Phase an einer Skala abgelesen. Die Ergebnisse sind in Tabelle 1 aufgeführt. Es zeigte sich, dass die Hydrophobierung unter Verdrängung der wässrigen Porenflüssigkeit nach einer Stunde abgeschlossen war.

b) In einer verschlossenen Schraubflasche wurden 50 g Gel und 100 ml HMDSO bei 50 °C mit 5,0 g konzentrierte Salzsäure versetzt, geschüttelt und die Menge der aus dem Gel verdrängten wässrigen Phase an einer Skala abgelesen. Die Ergebnisse sind in Tabelle 1 aufgeführt. Es zeigte sich, dass die Hydrophobierung unter Verdrängung der wässrigen Porenflüssigkeit nach einer Stunde abgeschlossen war.

c) In einer verschlossenen Schraubflasche wurden 50 g Gel und 100 ml einer HMDSO-Lösung (50 Gew.-% in n-Hexan) bei 50 °C mit 5,0 g Trimethylchlorsilan versetzt, geschüttelt und die Menge der aus dem Gel verdrängten wässrigen Phase an einer Skala abgelesen. Es zeigte sich, dass die Hydrophobierung unter Verdrängung der wässrigen Porenflüssigkeit nach zwei Stunden abgeschlossen war.

**Beispiel 6:**

In einem Becherglas wurden 150,0 g Wasser, 75,0 g Wasserglas und 75,0 g Kaliummethylsiliconat gemischt und in einem Eisbad auf 10 °C gekühlt.
In einer Schraubflasche wurden 200 g Salzsäure (7,5 Gew.-%) vorgelegt, in einem Eisbad auf unter 10 °C gekühlt und mit einem Magnetrührer bei 500 UpM gerührt.
Die gekühlte Wasserglas-Kaliummethylsiliconat-Lösung wurde über einen Tropftrichter langsam unter Rühren zu der Salzsäurelösung gegeben. Bei der Dosierung wurde darauf geachtet, dass die Temperatur nicht über 10 °C steigt. Bei einem pH-Wert von 5,3 wurde die Zugabe gestoppt und das Reaktionsgemisch auf Raumtemperatur erwärmt, wodurch Gelbildung stattfand. Für die Alterung wurde das erhaltene Gel in einem geschlossenen Gefäß für 3 Stunden bei 60 °C in einem Trockenschrank inkubiert. Danach wurde das Gel durch ein Sieb mit einer Maschenweite von 5 mm gedrückt, um Stücke kleiner 5 mm zu erhalten. Zur Entfernung der Salze wurden die Gelstücke dreimal für jeweils 12 Stunden in 60 °C heißem Wasser inkubiert (300 ml Wasser je 100 g Gel). Das Wasser wurde dabei nach jeweils 12 Stunden durch Dekantieren abgetrennt und anschließend durch frisches Wasser ersetzt. Vor der Oberflächenmodifizierung wurden 100 g des feuchten Gels mit 200 ml einer Ethanol-Wasser-Mischung (50 Gew.-% Ethanol) überschichtet und für 16 Stunden bei Raumtemperatur in einem geschlossenen Gefäß inkubiert. Anschließend wurde das Gel mittels Filtration über einen Büchner Trichter (Whatman® Filter, 125 mm, Grade 40) abgetrennt. Für die Oberflächenmodifizierung wurden die erhaltenen Gelstücke in einer verschlossenen Schraubflasche mit 200 ml HMDSO und 10,0 g Trimethylchlorsilan versetzt, geschüttelt und bei 60 °C für 4 Stunden in einem Trockenschrank inkubiert. Anschließend wurden die Gelstücke mittels Filtration über einen Büchner Trichter (Whatman® Filter, 125 mm, Grade 40) abgetrennt und in einem Vakuumtrockenschrank bei vermindertem Druck bis zur Gewichtskonstanz getrocknet (10 mbar, 120 °C). Die folgenden Werte wurden wie in den Analysemethoden beschrieben, bestimmt.

Dichte: 0,11 g/cm$^3$
BET: 511 m$^2$/g

**Beispiel 7:**

In einem Becherglas wurden 108,8 g Wasser und 108,8 g Wasserglas gemischt und in einem Eisbad auf 10 °C gekühlt. In einem zweiten Becherglas wurden 11,6 g Wasser und 11,6 g Kaliummethylsiliconat gemischt und in einem Eisbad auf 10 °C gekühlt. In einer Schraubflasche wurden 200 g Salzsäure (7,5 Gew.-%) vorgelegt, in einem Eisbad auf unter 10 °C gekühlt und mit einem Magnetrührer bei 500 UpM gerührt.
Die gekühlte Wasserglas-Lösung wurde über einen Tropftrichter langsam unter Rühren zu der Salzsäurelösung gegeben. Bei der Dosierung wurde darauf geachtet, dass die Temperatur nicht über 10 °C steigt. Nach der Zugabe wurde das Reaktionsgemisch für zwei Stunden bei Raumtemperatur gerührt und vor der Zugabe der zweiten Komponente wieder auf unter 10 °C gekühlt. Anschließend wurde die gekühlte Kaliummethylsiliconat-Lösung über einen Tropftrichter langsam unter Rühren zugegeben, wobei bei der Dosierung darauf geachtet wurde, dass die die Temperatur nicht über 10 °C steigt. Anschließend wurde der Rührer entfernt und das Sol auf Raumtemperatur erwärmt, wodurch Gelbildung stattfand. Für die Alterung wurde das erhaltene Gel in einem geschlossenen Gefäß für 3 Stunden bei 60 °C in einem Trockenschrank inkubiert. Danach wurde das Gel durch ein Sieb mit einer Maschenweite von 5 mm gedrückt, um Stücke kleiner 5 mm zu erhalten. Zur Entfernung der Salze wurden die Gelstücke dreimal für jeweils 12 Stunden in 60 °C heißem Wasser inkubiert (300 ml Wasser je 100 g Gel). Das Wasser wurde dabei nach jeweils 12 Stunden durch Dekantieren abgetrennt und anschließend durch frisches Wasser ersetzt. Vor der Oberflächenmodifizierung wurden 100 g des feuchten Gels mit 200 ml einer Ethanol-Wasser-Mischung (50 Gew.-% Ethanol) überschichtet und für 16 Stunden bei Raumtemperatur in einem geschlossenen Gefäß inkubiert. Anschließend wurde das Gel mittels Filtration über einen Büchner Trichter (Whatman® Filter, 125 mm, Grade 40) abgetrennt. Für die Oberflächenmodifizierung wurden die erhaltenen Gelstücke in einer verschlossenen Schraubflasche mit 200 ml HMDSO und 10,0 g Trimethylchlorsilan versetzt, geschüttelt und bei 60 °C für 2 Stunden in einem Trockenschrank inkubiert. Anschließend wurden die Gelstücke mittels Filtration über einen Büchner Trichter (Whatman® Filter, 125 mm, Grade 40) abgetrennt und in einem Vakuumtrockenschrank bei vermindertem Druck bis zur Gewichtskonstanz getrocknet (10 mbar, 120 °C). Die folgenden Werte wurden wie in den Analysemethoden beschrieben, bestimmt.

Dichte: 0,11 g/cm$^3$
BET: 498 m$^2$/g
BJH-Porenvolumen: 3,25 cm$^3$/g
Mittlerer Porendurchmesser: 22,1 nm

**Beispiel 8:**

In einer Schraubflasche wurden 84 g Wasser und 0,2 ml einer wässrigen Salzsäure (1 M) vorgelegt und auf 60 °C erhitzt. Unter intensivem Rühren (Magnetrührer, 500 UpM) wurde eine Mischung aus 41,2 g TEOS und 2,40 g Dimethyldimethoxysilan (DMDMS) zugegeben und für 2 Stunden bei 60 °C gerührt. Das molare Verhältnis TEOS:DMDMS betrug 9:1. Anschließend wurde das Sol mit 5 ml einer 0,25 molaren Ammoniak-Lösung versetzt. Für die Alterung wurde das erhaltene Gel in einem geschlossenen Gefäß für 3 Stunden bei 60 °C in einem Trockenschrank inkubiert. Anschließend wurde das Gel in Stücke von ca. 10 mm zerteilt. 50 g der Gelstücke wurden mit 100 ml HMDSO überschichtet und bei 50 °C mit 5,0 g TMCS versetzt und in einer verschlossenen Schraubflasche für 2 Stunden bei 50 °C in einem Trockenschrank inkubiert. Anschließend wurden die Gelstücke durch Filtration mittels eines Büchner-Trichters (Whatman® Filter, 125 mm, Grade 40) abgetrennt und in einem Vakuumtrockenschrank (80 °C und 10 mbar) bis zur Gewichtskonstanz getrocknet. Die folgenden Werte wurden wie in den Analysemethoden beschrieben, bestimmt.

Dichte: 0,17 g/cm$^3$
BET-Oberfläche: 790 m$^2$/g

**Vergleichsbeispiel 1:** Ermittlung der Hydrophobierungs- bzw. Lösemittelaustauschgeschwindigkeiten ohne Grundhydrophobierung des Gels, unter Anwesenheit eines Phasenvermittlers

In einem Rundkolben wurden 451 g Wasser und 0,7 ml einer wässrigen Salzsäure (1 M) vorgelegt und auf 60 °C erhitzt. Unter intensivem Rühren wurden 208 g TEOS zugegeben und für 2 Stunden bei 50 °C gerührt. Nach dem Abkühlen auf Raumtemperatur wurde das Sol in einem Becherglas mit 25 ml einer 0,25 molaren Ammoniak-Lösung versetzt. Für die Alterung wurde das erhaltene Gel in einem geschlossenen Gefäß für 3 Stunden bei 60 °C in einem Trockenschrank inkubiert. Anschließend wurde das Gel durch ein Sieb mit einer Maschenweite von 5 mm gedrückt, um Stücke kleiner 5 mm zu erhalten. Die Gel-Stücke wurden anschließend entsprechend der Varianten a) und b) umgesetzt.

a) In einer verschlossenen Schraubflasche wurden 50 g Gel und 100 ml HMDSO bei 50 °C mit 5,0 g Trimethylchlorsilan versetzt, geschüttelt und die Menge der aus dem Gel verdrängten wässrigen Phase an einer Skala abgelesen. Die Ergebnisse sind in Tabelle 1 aufgeführt. Es zeigte sich, dass die Hydrophobierung unter Verdrängung der wässrigen Porenflüssigkeit nach zwei Stunden abgeschlossen war.

b) In einer verschlossenen Schraubflasche wurden 50 g Gel und 100 ml HMDSO bei 50 °C mit 5,0 g konzentrierte Salzsäure versetzt, geschüttelt und die Menge der aus dem Gel verdrängten wässrigen Phase an einer Skala abgelesen. Die Ergebnisse sind in Tabelle 1 aufgeführt. Es zeigte sich, dass die Hydrophobierung unter Verdrängung der wässrigen Porenflüssigkeit nach drei Stunden abgeschlossen war.

**Vergleichsbeispiel 2:** Ermittlung der Hydrophobierungs- bzw. Lösemittelaustauschgeschwindigkeiten mit Grundhydrophobierung des Gels, unter Ausschluss eines Phasenvermittlers

Die Gelstücke kleiner 5 mm wurden wie unter Beispiel 5 beschrieben hergestellt. Statt der Silylierung entsprechend der Varianten a, b oder c wurden die Gelstücke zur Entfernung des Ethanols fünfmal für jeweils 24 Stunden in 60 °C heißem, destilliertem Wasser inkubiert (300 ml Wasser je 100 g Gel). Das Wasser wurde dabei nach jeweils 24 Stunden durch Dekantieren abgetrennt und anschließend durch frisches Wasser ersetzt. Die Gelstücke wurden anschließend entsprechend der Varianten a und b umgesetzt.

a) In einer verschlossenen Schraubflasche wurden 50 g Gel und 100 ml HMDSO bei 50 °C mit 5,0 g Trimethylchlorsilan versetzt, geschüttelt und die Menge der aus dem Gel verdrängten wässrigen Phase an einer Skala abgelesen. Die Ergebnisse sind in Tabelle 1 aufgeführt. Es zeigte sich, dass nach 24 Stunden 85 Vol.-% der zu erwartenden Menge an Porenflüssigkeit aus dem Gel verdrängt wurden.

b) In einer verschlossenen Schraubflasche wurden 50 g Gel und 100 ml HMDSO bei 50 °C mit 5,0 g konzentrierte Salzsäure versetzt, geschüttelt und die Menge der aus dem Gel verdrängten wässrigen Phase an einer Skala abgelesen. Die Ergebnisse sind in Tabelle 1 aufgeführt. Es zeigte sich, dass nach 24 Stunden 70 Vol.-% der zu erwartenden Menge an Porenflüssigkeit aus dem Gel verdrängt wurden.

**Vergleichsbeispiel 3:** Ermittlung der Hydrophobierungs- bzw. Lösemittelaustauschgeschwindigkeiten ohne Grundhydrophobierung des Gels, unter Ausschluss eines Phasenvermittlers

Die Gelstücke kleiner 5 mm wurden wie unter Vergleichsbeispiel 1 beschrieben hergestellt. Statt der Silylierung entsprechend der Varianten a, b oder c wurden die Gelstücke zur Entfernung des Ethanols fünfmal für jeweils 24 Stunden in destilliertem Wasser bei 60 °C inkubiert (300 ml Wasser je 100 g Gel). Das Wasser wurde dabei nach jeweils 24 Stunden durch Dekantieren abgetrennt und anschließend durch frisches Wasser ersetzt. Die Gelstücke wurden anschließend entsprechend der Varianten a und b umgesetzt.

a) In einer verschlossenen Schraubflasche wurden 50 g Gel und 100 ml HMDSO bei 50 °C mit 5,0 g Trimethylchlorsilan versetzt, geschüttelt und die Menge der aus dem Gel verdrängten wässrigen Phase an einer Skala abgelesen. Über einen Zeitraum von 24 Stunden fand keine Reaktion in Form einer Verdrängung der Porenflüssigkeit statt.

b) In einer verschlossenen Schraubflasche wurden 50 g Gel und 100 ml HMDSO bei 50 °C mit 5,0 g konzentrierte Salzsäure versetzt, geschüttelt und die Menge der aus dem Gel verdrängten wässrigen Phase an einer Skala abgelesen. Über einen Zeitraum von 24 Stunden fand keine Reaktion in Form einer Verdrängung der Porenflüssigkeit statt.

**Vergleichsbeispiel 4:** Ermittlung der Hydrophobierungs- bzw. Lösemittelaustauschgeschwindigkeiten bei gleicher Menge an Trimethylchlorsilan wie in Beispiel 5a, Vergleichsbeispielen 1a, 2a und 3a unter Substitution von Hexamethyldisiloxan durch n-Hexan

a) In einer verschlossenen Schraubflasche wurden 50 g gealtertes und zerkleinertes Gel hergestellt nach Beispiel 5 und 100 ml n-Hexan bei 50 °C mit 5,0 g Trimethylchlorsilan versetzt, geschüttelt und die Menge der aus dem Gel verdrängten wässrigen Phase an einer Skala abgelesen. Über einen Zeitraum von 24 Stunden fand keine Reaktion in Form einer Verdrängung der Porenflüssigkeit statt.

b) In einer verschlossenen Schraubflasche wurden 50 g gealtertes und zerkleinertes Gel hergestellt nach Vergleichsbeispiel 1 und 100 ml n-Hexan bei 50 °C mit 5,0 g Trimethylchlorsilan versetzt, geschüttelt und die Menge der aus dem Gel verdrängten wässrigen Phase an einer Skala abgelesen. Über einen Zeitraum von 24 Stunden fand keine Reaktion in Form einer Verdrängung der Porenflüssigkeit statt.

c) In einer verschlossenen Schraubflasche wurden 50 g gewaschenes Gel hergestellt nach Vergleichsbeispiel 2 und 100 ml n-Hexan bei 50 °C mit 5,0 g Trimethylchlorsilan versetzt, geschüttelt und die Menge der aus dem Gel verdrängten wässrigen Phase an einer Skala abgelesen. Über einen Zeitraum von 24 Stunden fand keine Reaktion in Form einer Verdrängung der Porenflüssigkeit statt.

d) In einer verschlossenen Schraubflasche wurden 50 g gewaschenes Gel hergestellt nach Vergleichsbeispiel 3 und 100 ml n-Hexan bei 50 °C mit 5,0 g Trimethylchlorsilan versetzt, geschüttelt und die Menge der aus dem Gel verdrängten wässrigen Phase an einer Skala abgelesen. Über einen Zeitraum von 24 Stunden fand keine Reaktion in Form einer Verdrängung der Porenflüssigkeit statt.

**Beispiel 9:**

In einem Becherglas wurden 80,0 g Wasserglas und 65,0 g Wasser gemischt und in einem Eisbad auf 10 °C gekühlt.
In einer Schraubflasche wurden 100 g Salzsäure (7,5 Gew.-%) vorgelegt, in einem Eisbad auf unter 10 °C gekühlt und mit einem Magnetrührer bei 500 UpM gerührt.
Die gekühlte Wasserglas-Lösung wurde über einen Tropftrichter langsam unter Rühren zu der Salzsäurelösung gegeben. Bei der Dosierung wurde darauf geachtet, dass die Temperatur nicht über 15 °C steigt. Bei einem pH-Wert von 2,5 wurde die Zugabe gestoppt. Anschließend wurden unter Rühren 6,25 g MTES zugegeben und das Gemisch aus dem Eisbad entfernt. Nachdem das Sol klar war, wurden 40,0 g Ethanol zugegeben. Schließlich wurde durch Zugabe einer 0,5 molaren Ammoniaklösung ein pH-Wert von 6,0 eingestellt, wodurch Gelbildung stattfand. Für die Alterung wurde das erhaltene Gel in einem geschlossenen Gefäß für 3 Stunden bei 60 °C in einem Trockenschrank inkubiert. Danach wurde das Gel durch ein Sieb mit einer Maschenweite von 5 mm gedrückt.
Für die Oberflächenmodifizierung wurden 100 g der erhaltenen Gelstücke in einer verschlossenen Schraubflasche mit 200 ml HMDSO, 10,0 g Salzsäure (32%ig) und 10 g Ethanol versetzt, geschüttelt und bei 70 °C für 4 Stunden in einem Trockenschrank inkubiert und dabei ca. alle 15 min das Reaktionsgefäß geschüttelt. Anschließend wurden die Gelstücke mittels Filtration abgetrennt, mit Wasser gewaschen und in einem Vakuumtrockenschrank bei vermindertem Druck bis zur Gewichtskonstanz getrocknet (10 mbar, 120 °C). Die folgenden Werte wurden wie in den Analysemethoden beschrieben, bestimmt.

Schüttdichte: 0,09 g/cm$^3$
BET: 790 m$^2$/g

**Beispiel 10:**

In einem Becherglas wurden 82,0 g Wasserglas, 5,0 g Kaliummethylsiliconat und 50,0 g Wasser gemischt und auf 10 °C gekühlt. In einer Schraubflasche wurden 100 g Salzsäure (7,5 Gew.-%) vorgelegt, in einem Eisbad auf unter 10 °C gekühlt und mit einem Magnetrührer bei 500 UpM gerührt.
Die gekühlte Wasserglas-Kaliummethylsiliconat-Lösung wurde über einen Tropftrichter langsam unter Rühren zu der Salzsäurelösung gegeben. Bei der Dosierung wurde darauf geachtet, dass die Temperatur nicht über 15 °C steigt. Bei einem pH-Wert von 1,5 wurde die Zugabe gestoppt. Anschließend wurden unter Rühren 100,0 g des Phasenvermittlers zugegeben (85 g Ethanol (a), 100 g Methanol (b), 100 g Isopropanol (c)) und das Gemisch aus dem Eisbad entfernt.

Schließlich wurde durch Zugabe einer 0,25 molaren Ammoniaklösung ein pH-Wert von 5,5 eingestellt, wodurch die Gelbildung stattfand. Für die Alterung wurde das erhaltene Gel in einem geschlossenen Gefäß für 3 Stunden bei 60 °C in einem Trockenschrank inkubiert. Danach wurde das Gel durch ein Sieb mit einer Maschenweite von 5 mm gedrückt, um Stücke kleiner 5 mm zu erhalten.

Für die Oberflächenmodifizierung wurden 100 g der erhaltenen Gelstücke in einer verschlossenen Schraubflasche mit 200 ml HMDSO und 10,0 g Salzsäure (32%ig) versetzt, geschüttelt und bei 70 °C für 4 Stunden in einem Trockenschrank inkubiert und dabei ca. alle 15 min das Reaktionsgefäß geschüttelt. Anschließend wurden die Gelstücke mittels Filtration abgetrennt, mit Wasser gewaschen und in einem Vakuumtrockenschrank bei vermindertem Druck bis zur Gewichtskonstanz getrocknet (10 mbar, 120 °C). Die folgenden Werte wurden wie in den Analysemethoden beschrieben, bestimmt.

a) Schüttdichte: 0,09 g/cm$^3$
b) Schüttdichte: 0,06 g/cm$^3$
c) Schüttdichte: 0,06 g/cm$^3$

**Beispiel 11:**

In einem Becherglas wurden 115 g Wasser und 1,5 g einer wässrigen Salzsäure (1 M) vorgelegt. Unter Rühren wurde eine Mischung aus 47,5 g TEOS und 1,4 g Trimethylethoxysilan zugegeben und für 16 Stunden bei Raumtemperatur gerührt.

Für die Gelbildung wurde das Sol mit 5,0 g einer 0,5 molaren Ammoniak-Lösung versetzt. Für die Alterung wurde das erhaltene Gel in einem geschlossenen Gefäß für 3 Stunden bei 60 °C in einem Trockenschrank inkubiert. Anschließend wurde das Gel in Stücke von ca. 5 mm zerteilt.

Für die Oberflächenmodifizierung wurden 100 g der erhaltenen Gelstücke in einer verschlossenen Schraubflasche mit 200 ml HMDSO, 10,0 g Salzsäure (32%ig) und 10 g Ethanol versetzt, geschüttelt und bei 70 °C für 3 Stunden in einem Trockenschrank inkubiert und dabei ca. alle 15 min das Reaktionsgefäß geschüttelt. Anschließend wurden die Gelstücke mittels Filtration abgetrennt, mit Wasser gewaschen und in einem Vakuumtrockenschrank bei vermindertem Druck bis zur Gewichtskonstanz getrocknet (10 mbar, 120 °C). Die folgenden Werte wurden wie in den Analysemethoden beschrieben, bestimmt.

Schüttdichte: 0,11 g/cm$^3$
BET: 715 m$^2$/g

Tabelle 1:

| Zeit [h] | Menge verdrängter Porenflüssigkeit [ml] | | | | | |
|---|---|---|---|---|---|---|
| | Beispiel | | Vergleichsbeispiel | | | |
| | 5a | 5b | 1a | 1b | 2a | 2b |
| 0,00 | 0,00 | 0,00 | 0,00 | 0,00 | 0,00 | 0,00 |
| 0,08 | 0,00 | 0,00 | nd | nd | nd | nd |
| 0,33 | 28,00 | 0,00 | nd | nd | nd | nd |
| 0,50 | nd | 0,00 | 0,00 | nd | nd | nd |
| 0,67 | nd | 24,00 | nd | nd | nd | nd |
| 0,83 | nd | nd | 13,00 | 0,00 | nd | nd |
| 1,00 | nd | nd | nd | nd | 0,00 | 0,00 |
| 1,17 | 49,00 | 47,00 | nd | nd | nd | nd |
| 1,20 | nd | nd | 31,00 | 0,00 | nd | nd |
| 1,50 | 49,00 | nd | nd | nd | nd | nd |
| 2,00 | nd | 49,00 | nd | nd | 0,00 | 0,00 |
| 2,10 | nd | nd | nd | 0,00 | nd | nd |
| 2,20 | nd | nd | nd | 19,00 | nd | nd |

(fortgesetzt)

| Zeit [h] | Menge verdrängter Porenflüssigkeit [ml] | | | | | |
|---|---|---|---|---|---|---|
| | Beispiel | | Vergleichsbeispiel | | | |
| | 5a | 5b | 1a | 1b | 2a | 2b |
| 2,40 | nd | nd | 49,00 | 38,00 | nd | nd |
| 3,00 | nd | nd | nd | nd | 15,00 | 0,00 |
| 3,20 | nd | nd | 49,00 | 50,00 | nd | nd |
| 6,00 | nd | nd | nd | nd | 28,00 | 0,00 |
| 24,00 | nd | nd | nd | nd | 42,00 | 35,00 |
| nd= der Wert wurde nicht gemessen | | | | | | |

## Patentansprüche

1. Verfahren zur Herstellung organisch modifizierter Aerogele, indem

 i) ein Sol enthaltend $[SiO_{4/2}]$ -Einheiten und $[R_xSiO_{(4-x)/2}]$-Einheiten , hergestellt wird,
 wobei bereits bei der Herstellung des Sols ein Phasenvermittler zugegeben oder ein Phasenvermittler aus dem Sol gebildet wird,
 ii) aus dem Sol ein Gel gebildet wird,
 wobei der Gehalt an Phasenvermittler in der Porenflüssigkeit zwischen 1 und 50 Gew.-% liegt,
 iii) das erhaltene Gel in Anwesenheit von über 0,1 Gew.-% des Phasenvermittlers in einem Gemisch enthaltend ein Disiloxan der Formel $R_3Si-O-SiR_3$ und Initiator oberflächenmodifiziert wird,
 wobei das Gemisch mindestens 20 Gew.-% des Disiloxans der Formel $R_3Si-O-SiR_3$ enthält,
 und wobei der Initiator aus Säure oder Chlorsilan oder Gemischen daraus besteht
 iv) und die erhaltenen Gele getrocknet werden,

 wobei X gleich oder verschieden sein kann und 1, 2 oder 3 ist und
 wobei R gleich oder verschieden sein kann und Wasserstoff oder ein organischer, substituierter oder unsubstituierter Rest ist.

2. Verfahren nach Anspruch 1, **dadurch gekennzeichnet, dass** im Schritt der Solherstellung (Schritt i) mindestens 1 Gew.% der Ausgangsstoffe zur Bildung von $[R_xSiO_{(4-x)/2}]$ -Einheiten erst später zu den bereits vorgelegten Ausgangsstoffen gegeben werden.

3. Verfahren nach einem oder mehreren der Ansprüche 1 oder 2, **dadurch gekennzeichnet, dass** Schritt iii ohne vorhergehenden Lösungsmittelaustausch stattfindet.

4. Verfahren nach einem oder mehreren der Ansprüche 1 bis 3, **dadurch gekennzeichnet, dass** der Initiator aus Trimethylchlorsilan oder Salzsäure oder Gemischen daraus besteht.

5. Verfahren nach einem oder mehreren der Ansprüche 1 bis 4, wobei gleichzeitig mit der Gelbildung eine Formgebung erfolgt.

6. Verfahren nach einem oder mehreren der Ansprüche 1 bis 5, **dadurch gekennzeichnet, dass** als Disiloxan Hexamethyldisiloxan eingesetzt wird.

7. Verfahren nach einem oder mehreren der Ansprüche 1 bis 5, **dadurch gekennzeichnet, dass** die Formgebung durch Dispergieren des Sols in einer kontinuierlichen Phase erfolgt, wobei die kontinuierliche Phase mindestens 20 Gew.-% des Disiloxans enthält und diese gleichzeitig als Reagenz zur Oberflächenmodifizierung dient.

8. Verfahren nach einem oder mehreren der Ansprüche 1 bis 7, **dadurch gekennzeichnet, dass** als Phasenvermittler Alkohole verwendet werden.

9. Verfahren nach Anspruch 8, **dadurch gekennzeichnet, dass** als Phasenvermittler Ethanol verwendet wird.

10. Verfahren nach einem oder mehreren der Ansprüche 1 bis 9, **dadurch gekennzeichnet, dass** höchstens 20 g Initiator pro 100 g Gel eingesetzt werden.

11. Verfahren nach einem oder mehreren der Ansprüche 1 bis 10, **dadurch gekennzeichnet, dass** als $[SiO_{4/2}]$-Ausgangsstoff Tetraethoxysilan (TEOS) und als $[R_xSiO_{(4-x)/2}]$-Ausgangsstoff Methyltriethoxysilan (MTES) oder deren Hydrolyseprodukte eingesetzt werden.

**Claims**

1. Process for producing organically modified aerogels by

   i) preparing a sol comprising $[SiO_{4/2}]$ units and $[R_xSiO_{(4-x)/2}]$ units,
   wherein a compatibilizer is admixed in the course of the step of preparing the sol or a compatibilizer is formed out of the sol,
   ii) forming a gel out of the sol,
   wherein the compatibilizer content of the pore liquid is between 1 and 50 wt%,
   iii) surface modifying the resultant gel in the presence of above 0.1 wt% of the compatibilizer in a mixture comprising a disiloxane of the formula $R_3Si-O-SiR_3$ and initiator, wherein the mixture comprises not less than 20 wt% of the disiloxane of the formula $R_3Si-O-SiR_3$,
   and wherein the initiator consists of an acid or a chlorosilane or mixtures thereof,
   iv) and drying the gels obtained,

   wherein each X is 1, 2 or 3 and may be the same or different, and
   wherein each R is hydrogen or a substituted or unsubstituted organic moiety and may be the same or different.

2. Process according to Claim 1, **characterized in that** the step of preparing the sol (step i) is carried out by later admixing not less than 1 wt% of the starting materials to form $[R_xSiO_{(4-x)/2}]$ units to the already initially charged starting materials.

3. Process according to one or more of Claims 1 or 2, **characterized in that** step iii takes place without a solvent exchange beforehand.

4. Process according to one or more of Claims 1 to 3, **characterized in that** the initiator consists of trimethylchlorosilane or hydrochloric acid or mixtures thereof.

5. Process according to one or more of Claims 1 to 4 wherein shaping is effected concurrently with the step of gel formation.

6. Process according to one or more of Claims 1 to 5, **characterized in that** the disiloxane used is hexamethyldisiloxane.

7. Process according to one or more of Claims 1 to 5, **characterized in that** said shaping is effected by dispersing the sol in a continuous phase, wherein the continuous phase comprises not less than 20 wt% of the disiloxane and simultaneously serves as reagent for surface modification.

8. Process according to one or more of Claims 1 to 7, **characterized in that** alcohols are used as compatibilizer.

9. Process according to Claim 8, **characterized in that** ethanol is used as compatibilizer.

10. Process according to one or more of Claims 1 to 9, **characterized in that** not more than 20 g of initiator are used per 100 g of gel.

11. Process according to one or more of Claims 1 to 10, **characterized in that** the $[SiO_{4/2}]$ starting material used is tetraethoxysilane (TEOS) and the $[R_xSio_{(4-x)/2}]$ starting material used is methyltriethoxysilane (MTES) or the hydrolysis products thereof.

**Revendications**

1. Procédé de fabrication d'aérogels organiquement modifiés, où

    i) on fabrique des unités [SiO$_{4/2}$] et [R$_x$SiO$_{(4-x)/2}$] contenant un sol,
    où, dès la fabrication du sol, on ajoute un intermédiaire de phase ou un intermédiaire de phase se forme à partir du sol,
    ii) un gel se forme à partir du sol,
    où la teneur en intermédiaire de phase dans le liquide de pores se situe entre 1 et 50 % en poids,
    iii) le gel obtenu est modifié en surface en présence de plus de 0,1 % en poids de l'intermédiaire de phase dans un mélange contenant un disiloxane de formule R$_3$Si-O-SiR$_3$ et un initiateur,
    le mélange contenant au moins 20 % en poids du disiloxane de formule R$_3$Si-O-SiR$_3$,
    et l'initiateur étant constitué d'acide ou de chlorosilane ou d'un de leurs mélanges, et
    iv) les gels obtenus sont séchés,

    où X peut être identique ou différent et est égal à 1, 2 ou 3, et
    où R peut être identique ou différent et est un hydrogène ou un résidu organique, substitué ou non substitué.

2. Procédé selon la revendication 1, **caractérisé en ce qu'**à l'étape de fabrication du sol (étape i), on n'ajoute au moins 1 % en poids de la substance initiale pour fabriquer des unités [R$_x$SiO$_{(4-x)/2}$] qu'après les substances initiales déjà introduites.

3. Procédé selon une ou plusieurs des revendications 1 ou 2, **caractérisé en ce que** l'étape iii a lieu sans changement préalable de solvant.

4. Procédé selon une ou plusieurs des revendications 1 à 3, **caractérisé en ce que** l'initiateur est constitué de triméthylchlorosilane ou d'acide chlorhydrique ou de leurs mélanges.

5. Procédé selon une ou plusieurs des revendications 1 à 4, où une mise en forme a lieu en même temps que la formation du gel.

6. Procédé selon une ou plusieurs des revendications 1 à 5, **caractérisé en ce qu'**on utilise de l'hexaméthyldisiloxane comme disiloxane.

7. Procédé selon une ou plusieurs des revendications 1 à 5, **caractérisé en ce que** la mise en forme se fait par dispersion du sol dans une phase continue, la phase continue contenant au moins 20 % en poids de disiloxane, et celle-ci sert en même temps de réactif de modification de surface.

8. Procédé selon une ou plusieurs des revendications 1 à 7, **caractérisé en ce qu'**on utilise comme intermédiaires de phase des alcools.

9. Procédé selon la revendication 8, **caractérisé en ce qu'**on utilise comme intermédiaire de phase de l'éthanol.

10. Procédé selon une ou plusieurs des revendications 1 à 9, **caractérisé en ce qu'**on utilise au maximum 20 g d'initiateur pour 100 g de gel.

11. Procédé selon une ou plusieurs des revendications 1 à 10, **caractérisé en ce qu'**on utilise comme matière première de [SiO$_{4/2}$] du tetraéthoxysilane (TEOS) et comme matière première de [R$_x$SiO$_{(4-x)/2}$] du méthyltriéthoxysilane (MTES) ou leurs produits d'hydrolyse.

Abbildung 1:

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0948395 B1
- CN 101691227
- CN 102897779
- CN 10897779

- EP 0948395 A
- US 6481649 B1
- US 6620355 B1

**In der Beschreibung aufgeführte Nicht-Patentliteratur**

- **M. SCHMIDT.** *Journal of non-Crystalline Solids,* 1998, vol. 225, 24-29
- *Journal of non-Crystalline Solids,* 1998, vol. 225, 24-29

- **Z. SHAO ; F. LUO ; X. CHENG ; Y. ZHANG.** *Material Chemistry and Physics,* 2013, vol. 141, 570-575